# EUROPEAN PATENT APPLICATION

(11) **EP 4 751 715 A1**
(43) Date of publication of application: **03.06.2026**
(21) Application number: 24844862.3
(22) Date of filing: 26.07.2024
(51) Int. Cl.: A61K 9/10, A61K 31/445, A61K 47/10, A61K 47/28, A61K 9/16, A61P 23/00, A61P 29/00

(54) **COMPOSITION OF ROPIVACAINE OR PHARMACEUTICALLY ACCEPTABLE SALT OF ROPIVACAINE, AND USE OF COMPOSITION**

(30) Priority: 27.07.2023 CN 202310933637
(71) Applicant: Nanjing Delova Biotech Co. Ltd., Nanjing, Jiangsu 210000 (CN)
(72) Inventor: WANG, Qingsong, Nanjing, Jiangsu 210000 (CN); LI, Ling, Nanjing, Jiangsu 210000 (CN); WU, Qu, Nanjing, Jiangsu 210000 (CN); YANG, Yibo, Nanjing, Jiangsu 210000 (CN)
(74) Representative: Gille Hrabal Partnerschaftsgesellschaft mbB Patentanwälte
(86) International application number: PCT/CN2024/107691
(87) International publication number: WO 2025/021176

(57) **Abstract**

Provided are a composition of ropivacaine or a pharmaceutically acceptable salt of ropivacaine, and a use of a composition. A suspension and a solid composition can ensure a sterility level by means of sterilization and filtration, and the obtained composition has good stability and controllable product quality, is easily administered, causes little irritation, and has good medication safety and tolerance.

## Description

The present application claims priority to:
Chinese Patent Application No. 202310933637.5 filed with China National Intellectual Property Administration on July 27, 2023 and entitled "COMPOSITION OF ROPIVACAINE OR PHARMACEUTICALLY ACCEPTABLE SALT OF ROPIVACAINE, AND USE OF COMPOSITION", which is incorporated herein by reference in its entirety.

### TECHNICAL FIELD

The present disclosure relates to the field of pharmaceutical formulations, and particularly to a composition of ropivacaine or a pharmaceutically acceptable salt thereof, especially a long-acting suspension or solid composition of ropivacaine or a pharmaceutically acceptable salt thereof.

### BACKGROUND

Postoperative pain is acute, moderate-to-severe pain that occurs immediately after surgery and generally lasts no more than 7 days. Reports show that about 75% of surgical patients experience postoperative pain, and the pain of more than 50% of these patients cannot be adequately relieved. Such insufficient control of postoperative pain may lead to adverse physiological reactions in patients after surgery and increase risks such as chronic postoperative pain.

In order to cope with the aforementioned risks associated with postoperative pain, patients are usually required to take analgesic drugs in clinical practice. Such drugs mainly include opioid analgesics represented by morphine, non-steroidal anti-inflammatory drugs represented by aspirin, local anesthetics represented by bupivacaine, etc. Currently, opioid analgesics account for the majority share of the analgesic market. Although their analgesic effects are strong, they are prone to causing adverse effects in various systems throughout the body. According to statistics from the U.S. Centers for Disease Control and Prevention, as of 2017, approximately 15 per 100,000 deaths in the United States were associated with opioid overdoses.

The clinical consensus on postoperative management in China is to reduce the use of opioids and treat surgical pain with multimodal analgesia, but deficiencies still exist. For example, using local anesthetics to treat postoperative pain can avoid the adverse effects of opioids; however, the local anesthetics currently used generally have a short duration of action, with efficacy only maintained for several hours after a single administration, which fails to meet the needs of a treatment cycle for postoperative pain. Therefore, the clinical demand for long-acting non-opioid drugs for the treatment of postoperative pain remains unmet, and prolonging the effective analgesic duration of local anesthetics and reducing the use of opioids are current research hotspots.

Currently, all long-acting local anesthetics marketed outside of China use bupivacaine or salts thereof as the active ingredient, employing liposomes (Exparel^{®}), collagen implants (XaraColl^{®}), polyorthoester polymers (Zynrelef^{®}), and sucrose acetate isobutyrate (Posimir^{®}) as sustained-release platforms. However, these technologies face issues of complex production processes, insufficient analgesic duration, or excipient safety concerns. Moreover, the dose of bupivacaine is highly restricted due to its central nervous system and cardiovascular toxicity.

Compared with bupivacaine, ropivacaine of the following formula has advantages such as lower toxicity, higher safety, and higher tolerated dose. Therefore, it has been developed as a promising long-acting local anesthetic alternative to bupivacaine.

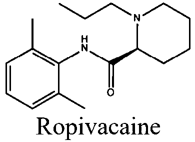

Currently existing long-acting ropivacaine formulations mainly include formulations based on oil sustained-release systems, lipid-based sustained-release systems, polymer-based sustained-release systems, suspension sustained-release systems, etc. However, the aforementioned systems still have the following defects:
The drug release process of oil sustained-release system formulations involves the dissolved drug molecules being partitioned from the oily formulation into the aqueous interstitial fluid and subsequently absorbed into the blood. Therefore, the sustained-release effect of the drug is closely related to the time required for the drug to be released from the formulation, and achieving a sustained-release duration of 3 days or longer is difficult with oil systems.

The preparation processes of lipid-based sustained-release systems and polymer-based microsphere sustained-release systems are often complex, difficult to achieve commercial production, and also face quality control issues. For sustained-release formulations containing polymers, there exists the issue of local accumulation due to the long degradation cycle of the polymers *in vivo.* Additionally, degradation products of PLGA-based sustained-release systems include glycolic acid and lactic acid, and the acidic degradation products may cause inflammatory reaction side effects at the site of administration, which is not conducive to treatment.

Suspension systems are currently all prepared by "top-down" methods, which involve pulverizing larger drug particles into the required particle size through mechanical means. Currently, the more commonly used top-down methods include wet grinding and jet milling, high-speed shearing, and high-pressure homogenization. However, such methods require the use of sterile starting materials and a sterile environment, and carry high sterile assurance risks, resulting in greater difficulty in production processes and higher costs.

Therefore, there is an urgent need to develop a long-acting ropivacaine formulation suitable for injection that exhibits favorable stability, safety, and/or tolerability. It is worth expecting that said long-acting ropivacaine formulation also possesses advantages such as easy large-scale production and controllable quality.

### SUMMARY

To overcome the defects described above, the present disclosure provides a suspension and a solid composition of ropivacaine or a pharmaceutically acceptable salt thereof, as well as preparation methods therefor and uses thereof, as described below. The present disclosure further provides a preparation method suitable for solid compositions of ropivacaine and other active pharmaceutical ingredients, and a solid composition obtained by the preparation method.

The present disclosure provides a suspension of ropivacaine or a pharmaceutically acceptable salt thereof, the suspension comprising:
a) ropivacaine or the pharmaceutically acceptable salt thereof,
   wherein in the suspension, ropivacaine or the pharmaceutically acceptable salt thereof is present in the form of particles, for example, in the form of crystals, preferably in the form of regular crystals, such as rod-shaped crystals or block-shaped crystals.

According to an embodiment of the present disclosure, in the suspension, ropivacaine or the pharmaceutically acceptable salt thereof has a D90 particle size of less than or equal to 200 µm, or less than or equal to 150 µm, or less than or equal to 120 µm, or less than or equal to 100 µm. Preferably, in the suspension, ropivacaine or the pharmaceutically acceptable salt thereof has a D90 particle size ranging from 10 µm to 90 µm, for example, 10 µm to 80 µm, preferably 10 µm to 70 µm, and more preferably 10 µm to 60 µm. As an example, in the suspension, ropivacaine or the pharmaceutically acceptable salt thereof may have a D90 particle size of 10 µm, 15 µm, 20 µm, 25 µm, 30 µm, 35 µm, 40 µm, 45 µm, 50 µm, 55 µm, or 60 µm.

According to an embodiment of the present disclosure, the pharmaceutically acceptable salt of ropivacaine is an acidic salt of ropivacaine, i.e., an addition salt of ropivacaine with a pharmaceutically acceptable acid. For example, the acidic salt of ropivacaine includes, but is not limited to, one or more selected from the following salts of ropivacaine: acetate, benzoate, benzenesulfonate, hydrobromide, camphorsulfonate, hydrochloride, citrate, edisylate, fumarate, glucoheptonate, gluconate, glucuronate, hydroiodide, isethionate, lactate, lactobionate, dodecylsulfate, malate, maleate, methanesulfonate, naphthoate, naphthalenesulfonate, nitrate, stearate, palmitate, myristate, laurate, oleate, oxalate, pamoate, phosphate, polygalacturonate, succinate, sulfate, sulfosalicylate, tartrate, toluenesulfonate, and trifluoroacetate.

It should be understood that in the context of this specification, the molar ratio of ropivacaine to the anion of the pharmaceutically acceptable acid is a ratio such that the pharmaceutically acceptable salt of ropivacaine is electrically neutral. For example, when the pharmaceutically acceptable acid is a monoprotic acid, the molar ratio of the acid to ropivacaine is 1:1; when the pharmaceutically acceptable acid is a diprotic acid, the molar ratio of the acid to ropivacaine is 1:2.

According to an embodiment of the present disclosure, the suspension optionally further comprises: b) a pharmaceutically acceptable stabilizer.

According to an embodiment of the present disclosure, the pharmaceutically acceptable stabilizer is selected from one or more of a long-chain alcohol (e.g., a C₁₈-C₃₀ long-chain alcohol) and a C₁₈-C₃₀ acid or a salt thereof.

According to an embodiment of the present disclosure, the pharmaceutically acceptable stabilizer is selected from one or more of cholesterol, octadecanol, cholic acid or a salt thereof, deoxycholic acid or a salt thereof, taurodeoxycholic acid or a salt thereof, ursodeoxycholic acid or a salt thereof, chenodeoxycholic acid or a salt thereof, and glycocholic acid or a salt thereof.

According to an embodiment of the present disclosure, the pharmaceutically acceptable stabilizer is selected from one or more of cholesterol, octadecanol, cholic acid, deoxycholic acid, taurodeoxycholic acid, ursodeoxycholic acid, chenodeoxycholic acid, glycocholic acid, sodium cholate, sodium deoxycholate, sodium taurodeoxycholate, sodium ursodeoxycholate, sodium chenodeoxycholate, and sodium glycocholate.

According to an embodiment of the present disclosure, the molar ratio of ropivacaine or the pharmaceutically acceptable salt thereof to the stabilizer ranges from 0.5:1 to 30:1, preferably 1:1 to 15:1, preferably 1:1 to 10:1, and more preferably 1:1 to 7:1, and more preferably is 1:1, 1.5:1, 2:1, 2.5:1, 3:1, 3.5:1, 4:1, 4.5:1, 5:1, 5.5:1, 6:1, 6.5:1, or 7:1.

According to an embodiment of the present disclosure, the suspension optionally further comprises: c) a pharmaceutically acceptable dispersant.

According to an embodiment of the present disclosure, the pharmaceutically acceptable dispersant is selected from one or more of a polysorbate (Tween), poloxamer, polyoxyethylene fatty acid ester, polyoxyethylene fatty acid ether, sucrose fatty acid ester, sodium dodecyl sulfate, macrogol 15 hydroxystearate (HS-15), and a phospholipid.

According to an embodiment of the present disclosure, the polysorbate is selected from one or more of polysorbate 80 (Tween 80) and polysorbate 20 (Tween 20).

According to an embodiment of the present disclosure, the phospholipid is selected from one or more of DMPC (dimyristoylphosphatidylcholine), DPPC (dipalmitoylphosphatidylcholine), DLPC (dilauroylphosphatidylcholine), SPC (soybean phosphatidylcholine), EPC (egg phosphatidylcholine), DEPC (dierucoylphosphatidylcholine), DOPC (dioleoylphosphatidylcholine), and POPC (palmitoyloleoylphosphatidylcholine).

According to an embodiment of the present disclosure, the molar ratio of ropivacaine or the pharmaceutically acceptable salt thereof to the pharmaceutically acceptable dispersant ranges from 50:1 to 1:1, preferably 40:1 to 2:1, more preferably 30:1 to 2:1, and more preferably 20:1 to 2:1, and more preferably is 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, or 2:1.

According to an embodiment of the present disclosure, the suspension optionally further comprises: d) a clinically acceptable diluent.

According to an embodiment of the present disclosure, the clinically acceptable diluent includes, but is not limited to, one or more of water for injection, a sodium chloride injection, a glucose injection, a Ringer's lactate solution, an aqueous sucrose solution, an aqueous xylitol solution, and a pH buffer. According to an embodiment of the present disclosure, the sodium chloride injection includes, but is not limited to, a 0.9% sodium chloride injection.

According to an embodiment of the present disclosure, the glucose injection includes, but is not limited to, a 5% glucose injection.

According to an embodiment of the present disclosure, the pH buffer is a buffer with a pH ranging from 5.0 to 7.4, which includes, but is not limited to, one or more of a phosphate buffer, a glycine buffer, a histidine buffer, a citrate buffer, and an acetate buffer.

According to an embodiment of the present disclosure, the suspension is a suspension injection.

According to an embodiment of the present disclosure, in the suspension, the concentration of ropivacaine or the pharmaceutically acceptable salt thereof ranges from about 0.1 mg/mL to about 300 mg/mL, preferably about 1 mg/mL to about 50 mg/mL, and more preferably about 2.5 mg/mL to about 40 mg/mL. As an example, in the suspension, the concentration of ropivacaine or the pharmaceutically acceptable salt thereof is about 0.1 mg/mL, 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, or 100 mg/mL. According to an embodiment of the present disclosure, the suspension optionally further comprises: e) a pharmaceutically acceptable inorganic salt.

According to an embodiment of the present disclosure, the pharmaceutically acceptable inorganic salt has a water solubility at 20 °C of greater than 10 g/100 mL.

According to an embodiment of the present disclosure, the pharmaceutically acceptable inorganic salt is selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride. According to an embodiment of the present disclosure, the molar ratio of ropivacaine or the pharmaceutically acceptable salt thereof to the pharmaceutically acceptable inorganic salt is ≥ 1:0.02, and preferably ranges from 1:0.02 to 1:2.0, and an example thereof is 1:0.02, 1:0.05, 1:0.1, 1:0.2, 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, or 1:2.0.

According to an embodiment of the present disclosure, the suspension optionally further comprises: f) a pharmaceutically acceptable pH regulator.

According to an embodiment of the present disclosure, the pH regulator is selected from an alkaline pH regulator and an acidic pH regulator.

According to an embodiment of the present disclosure, the alkaline pH regulator is selected from one or more of meglumine, tromethamine, sodium hydroxide, potassium hydroxide, sodium carbonate, sodium bicarbonate, magnesium hydroxide, calcium hydroxide, sodium phosphate, disodium hydrogen phosphate, potassium phosphate, dipotassium hydrogen phosphate, sodium citrate, sodium acetate, potassium acetate, ammonium acetate, triethylamine, ethanolamine, and diethanolamine, preferably one or more of sodium hydroxide, potassium hydroxide, magnesium hydroxide, sodium bicarbonate, and calcium hydroxide.

According to an embodiment of the present disclosure, the acidic pH regulator is selected from one or more of citric acid, tartaric acid, maleic acid, malic acid, fumaric acid, succinic acid, hydrochloric acid, phosphoric acid, ascorbic acid, lactic acid, acetic acid, methanesulfonic acid, oxalic acid, sulfuric acid, glycine, sodium dihydrogen phosphate, and potassium dihydrogen phosphate, preferably one or more of citric acid, tartaric acid, maleic acid, malic acid, fumaric acid, succinic acid, hydrochloric acid, phosphoric acid, lactic acid, and acetic acid.

According to an embodiment of the present disclosure, the pH value of the suspension ranges from 6.0 to 9.0, preferably 6.0 to 8.5, preferably 6.0 to 8.0, and more preferably 6.0 to 7.5, and more preferably is 6.2, 6.4, 6.6, 6.8, 7.0, 7.2, or 7.4.

According to an embodiment of the present disclosure, ropivacaine in the suspension may also be prepared *in situ,* for example, by reaction of a pharmaceutically acceptable salt of ropivacaine with a base, preferably by reaction of a pharmaceutically acceptable salt of ropivacaine with the alkaline pH regulator, wherein the pharmaceutically acceptable salt of ropivacaine has the definition described above.

According to an embodiment of the present disclosure, when ropivacaine is prepared *in situ* as described above, the molar ratio of the pharmaceutically acceptable salt of ropivacaine to the alkaline pH regulator may range from 1:0.5 to 1:2.0, preferably 1:0.8 to 1:1.5, and preferably 1: 1.0 to 1:1.2. As an example, the molar ratio of the pharmaceutically acceptable salt of ropivacaine to the alkaline pH regulator is 1:0.6, 1:0.7, 1:0.8, 1:0.9, 1:1.0, 1:1.1, 1:1.2, 1:1.3, 1:1.4, 1:1.5, 1:1.6, 1:1.7, 1:1.8, 1:1.9, or 1:2.0.

According to an embodiment of the present disclosure, the suspension optionally further comprises: g) a pharmaceutically acceptable osmotic pressure regulator.

According to an embodiment of the present disclosure, the osmotic pressure regulator is selected from one or more of sodium chloride, mannitol, lactose, glucose, sorbitol, and glycerol, preferably one or more of sodium chloride, glucose, sorbitol, and mannitol.

According to an embodiment of the present disclosure, the osmotic pressure of the suspension injection is comparable to the physiological osmotic pressure.

According to an embodiment of the present disclosure, the suspension optionally further comprises: h) a pharmaceutically acceptable lyoprotectant.

According to an embodiment of the present disclosure, the pharmaceutically acceptable lyoprotectant is selected from one or more of mannitol, lactose, trehalose, sucrose, maltose, dextrin, stachyose, gelatin, pectin, sugar alcohol, sorbitol, proline, histidine, lysine, arginine, polyethylene glycol, and polyvinylpyrrolidone, preferably one or more of mannitol, lactose, trehalose, sucrose, and polyvinylpyrrolidone.

According to an embodiment of the present disclosure, the suspension optionally does not comprise a pharmaceutically acceptable suspending agent. The suspending agent is preferably a water-soluble polymer compound, including but not limited to one or more of carboxymethyl cellulose or a sodium salt thereof, hydroxypropyl cellulose or a sodium salt thereof, hydroxypropyl methylcellulose or a sodium salt thereof, methyl cellulose or a sodium salt thereof, hydroxyethyl cellulose or a sodium salt thereof, sodium hyaluronate, and polyvinylpyrrolidone.

The present disclosure further provides a single-dose form or a multi-dose form of the suspension, wherein each dose unit of the single-dose form or the multi-dose form comprises ropivacaine or a pharmaceutically acceptable salt thereof equivalent to ropivacaine free base in an amount ranging from 5 mg to 1200 mg, preferably 20 mg to 1200 mg, and more preferably 40 mg to 1200 mg. Preferably, each dose unit of the single-dose form or the multi-dose form is contained in an individual container.

The present disclosure further provides a solid composition of ropivacaine or a pharmaceutically acceptable salt thereof, the solid composition comprising:
i) ropivacaine or the pharmaceutically acceptable salt thereof,
wherein in the solid composition, ropivacaine or the pharmaceutically acceptable salt thereof is present in the form of particles, for example, in the form of crystals, preferably in the form of regular crystals, such as rod-shaped or block-shaped crystals.

According to an embodiment of the present disclosure, in the solid composition, ropivacaine or the pharmaceutically acceptable salt thereof has a D90 particle size of less than or equal to 200 µm, or less than or equal to 150 µm, or less than or equal to 120 µm, or less than or equal to 100 µm. Preferably, in the suspension, ropivacaine or the pharmaceutically acceptable salt thereof has a D90 particle size ranging from 10 µm to 90 µm, for example, 10 µm to 80 µm, preferably 10 µm to 70 µm, and more preferably 10 µm to 60 µm. As an example, in the suspension, ropivacaine or the pharmaceutically acceptable salt thereof may have a D90 particle size of 10 µm, 15 µm, 20 µm, 25 µm, 30 µm, 35 µm, 40 µm, 45 µm, 50 µm, 55 µm, or 60 µm.

According to an embodiment of the present disclosure, the pharmaceutically acceptable salt of ropivacaine is an acidic salt of ropivacaine, i.e., an addition salt of ropivacaine with a pharmaceutically acceptable acid. For example, the acidic salt of ropivacaine includes, but is not limited to, one or more selected from the following salts of ropivacaine: acetate, benzoate, benzenesulfonate, hydrobromide, camphorsulfonate, hydrochloride, citrate, edisylate, fumarate, glucoheptonate, gluconate, glucuronate, hydroiodide, isethionate, lactate, lactobionate, dodecylsulfate, malate, maleate, methanesulfonate, naphthoate, naphthalenesulfonate, nitrate, stearate, palmitate, myristate, laurate, oleate, oxalate, pamoate, phosphate, polygalacturonate, succinate, sulfate, sulfosalicylate, tartrate, toluenesulfonate, and trifluoroacetate.

According to an embodiment of the present disclosure, the solid composition optionally further comprises:
ii) a pharmaceutically acceptable stabilizer.

According to an embodiment of the present disclosure, the pharmaceutically acceptable stabilizer is selected from one or more of a long-chain alcohol (e.g., a C₁₈-C₃₀ long-chain alcohol) and a C₁₈-C₃₀ acid or a salt thereof.

According to an embodiment of the present disclosure, the pharmaceutically acceptable stabilizer is selected from one or more of cholesterol, octadecanol, cholic acid or a salt thereof, deoxycholic acid or a salt thereof, taurodeoxycholic acid or a salt thereof, ursodeoxycholic acid or a salt thereof, chenodeoxycholic acid or a salt thereof, and glycocholic acid or a salt thereof.

According to an embodiment of the present disclosure, the pharmaceutically acceptable stabilizer is selected from one or more of cholesterol, octadecanol, cholic acid, deoxycholic acid, ursodeoxycholic acid, chenodeoxycholic acid, glycocholic acid, sodium cholate, sodium deoxycholate, and sodium taurodeoxycholate.

According to an embodiment of the present disclosure, the molar ratio of ropivacaine or the pharmaceutically acceptable salt thereof to the stabilizer ranges from 0.5:1 to 30:1, preferably 1:1 to 15:1, preferably 1:1 to 10:1, and more preferably 1:1 to 7:1, and more preferably is 1:1, 1.5:1, 2:1, 2.5:1, 3:1, 3.5:1, 4:1, 4.5:1, 5:1, 5.5:1, 6:1, 6.5:1, or 7:1.

According to an embodiment of the present disclosure, the solid composition optionally further comprises:
iii) a pharmaceutically acceptable dispersant.

According to an embodiment of the present disclosure, the pharmaceutically acceptable dispersant is selected from one or more of a polysorbate (Tween), poloxamer, polyoxyethylene fatty acid ester, polyoxyethylene fatty acid ether, sucrose fatty acid ester, sodium dodecyl sulfate, macrogol 15 hydroxystearate (HS-15), and a phospholipid.

According to an embodiment of the present disclosure, the polysorbate is selected from one or more of polysorbate 80 (Tween 80) and polysorbate 20 (Tween 20).

According to an embodiment of the present disclosure, the phospholipid is selected from one or more of DMPC (dimyristoylphosphatidylcholine), DPPC (dipalmitoylphosphatidylcholine), DLPC (dilauroylphosphatidylcholine), SPC (soybean phosphatidylcholine), EPC (egg phosphatidylcholine), DEPC (dierucoylphosphatidylcholine), DOPC (dioleoylphosphatidylcholine), and POPC (palmitoyloleoylphosphatidylcholine).

According to an embodiment of the present disclosure, the molar ratio of ropivacaine or the pharmaceutically acceptable salt thereof to the pharmaceutically acceptable dispersant ranges from 50:1 to 1:1, preferably 40:1 to 2:1, more preferably 30:1 to 2:1, and more preferably 20:1 to 2:1, and more preferably is 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, or 2:1.

According to an embodiment of the present disclosure, the solid composition optionally further comprises:
iv) a pharmaceutically acceptable inorganic salt.

According to an embodiment of the present disclosure, the pharmaceutically acceptable inorganic salt has a solubility in water at 20 °C of greater than 10 g/100 mL.

According to an embodiment of the present disclosure, the solid composition optionally comprises: an inorganic salt selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride.

According to an embodiment of the present disclosure, the molar ratio of ropivacaine or the pharmaceutically acceptable salt thereof to the pharmaceutically acceptable inorganic salt is ≥ 1:0.02, preferably ranges from 1:0.02 to 1:2.0, and more preferably is 1:0.02, 1:0.05, 1:0.1, 1:0.2, 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, or 1:2.0. According to an embodiment of the present disclosure, the solid composition optionally further comprises:
v) a pharmaceutically acceptable pH regulator.

According to an embodiment of the present disclosure, the pH regulator is selected from an alkaline pH regulator and an acidic pH regulator.

According to an embodiment of the present disclosure, the alkaline pH regulator is selected from one or more of meglumine, tromethamine, sodium hydroxide, potassium hydroxide, sodium carbonate, sodium bicarbonate, magnesium hydroxide, calcium hydroxide, sodium phosphate, disodium hydrogen phosphate, potassium phosphate, dipotassium hydrogen phosphate, sodium citrate, sodium acetate, potassium acetate, ammonium acetate, triethylamine, ethanolamine, and diethanolamine, preferably one or more of sodium hydroxide, potassium hydroxide, magnesium hydroxide, sodium bicarbonate, and calcium hydroxide.

According to an embodiment of the present disclosure, the acidic pH regulator is selected from one or more of citric acid, tartaric acid, maleic acid, malic acid, fumaric acid, succinic acid, hydrochloric acid, phosphoric acid, ascorbic acid, lactic acid, acetic acid, methanesulfonic acid, oxalic acid, sulfuric acid, glycine, sodium dihydrogen phosphate, and potassium dihydrogen phosphate, preferably one or more of citric acid, tartaric acid, maleic acid, malic acid, fumaric acid, succinic acid, hydrochloric acid, phosphoric acid, lactic acid, and acetic acid.

According to an embodiment of the present disclosure, the solid composition forms a suspension upon reconstitution.

According to an embodiment of the present disclosure, the pH value of the suspension ranges from 6.0 to 9.0, preferably 6.0 to 8.5, preferably 6.0 to 8.0, and more preferably 6.0 to 7.5, and more preferably is 6.2, 6.4, 6.6, 6.8, 7.0, 7.2, or 7.4.

According to an embodiment of the present disclosure, the solid composition optionally further comprises:
vi) a pharmaceutically acceptable osmotic pressure regulator.

According to an embodiment of the present disclosure, the pharmaceutically acceptable osmotic pressure regulator is selected from one or more of sodium chloride, mannitol, lactose, glucose, sorbitol, and glycerol, preferably one or more of sodium chloride, glucose, sorbitol, and mannitol. According to an embodiment of the present disclosure, the solid composition optionally further comprises:
vii) a pharmaceutically acceptable lyoprotectant.

According to an embodiment of the present disclosure, the pharmaceutically acceptable lyoprotectant is selected from one or more of mannitol, lactose, trehalose, sucrose, maltose, dextrin, stachyose, gelatin, pectin, sugar alcohol, sorbitol, proline, histidine, lysine, arginine, polyethylene glycol, and polyvinylpyrrolidone, preferably one or more of mannitol, lactose, trehalose, sucrose, and polyvinylpyrrolidone.

According to an embodiment of the present disclosure, the solid composition optionally does not comprise a pharmaceutically acceptable suspending agent, wherein the suspending agent is preferably a water-soluble polymer compound, including but not limited to one or more of carboxymethyl cellulose or a sodium salt thereof, hydroxypropyl cellulose or a sodium salt thereof, hydroxypropyl methylcellulose or a sodium salt thereof, methyl cellulose or a sodium salt thereof, hydroxyethyl cellulose or a sodium salt thereof, sodium hyaluronate, and polyvinylpyrrolidone.

The present disclosure further provides a preparation method for a solid pharmaceutical composition, the preparation method comprising: drying a solution comprising an active pharmaceutical ingredient to obtain the solid composition.

According to an embodiment of the present disclosure, in the solid pharmaceutical composition obtained after drying, the active pharmaceutical ingredient is present in the form of particles, for example, in the form of crystals, preferably in the form of regular crystals, such as rod-shaped crystals or block-shaped crystals.

According to an embodiment of the present disclosure, the preparation method comprises: sterilizing, filtering, and drying a solution comprising an active pharmaceutical ingredient to obtain the solid pharmaceutical composition.

According to an embodiment of the present disclosure, in the solid pharmaceutical composition obtained after drying, the active pharmaceutical ingredient has a D90 particle size of less than or equal to 200 µm, or less than or equal to 150 µm, or less than or equal to 120 µm, or less than or equal to 100 µm. Preferably, the active pharmaceutical ingredient has a D90 particle size ranging from 10 µm to 90 µm, for example, 10 µm to 80 µm, preferably 10 µm to 70 µm, and more preferably 10 µm to 60 µm. As an example, the active pharmaceutical ingredient may have a D90 particle size of 10 µm, 15 µm, 20 µm, 25 µm, 30 µm, 35 µm, 40 µm, 45 µm, 50 µm, 55 µm, or 60 µm.

According to an embodiment of the present disclosure, the drying is performed by a method including evaporation drying, freeze drying, and spray drying, preferably freeze drying.

According to an embodiment of the present disclosure, the freeze drying comprises a pre-freezing procedure and a drying procedure.

According to an embodiment of the present disclosure, the freeze drying may be performed in a container such as a vial.

According to an embodiment of the present disclosure, the active pharmaceutical ingredient may be dissolved in a solvent to form the solution comprising the active pharmaceutical ingredient.

According to an embodiment of the present disclosure, the solvent may be selected from an inorganic solvent, an organic solvent, and a mixture thereof suitable for dissolving the active pharmaceutical ingredient. For example, the solvent is water, an organic solvent, or a co-solvent system comprising water and an organic solvent, preferably one or more of water, methanol, ethanol, *n*-propanol, isopropanol, *n*-butanol, isobutanol, *tert*-butanol, acetone, acetonitrile, dichloromethane, and dimethyl sulfoxide, and more preferably water, ethanol, isopropanol, *tert*-butanol, or a co-solvent system comprising water and one or more organic solvents selected from the following: methanol, ethanol, *n*-propanol, isopropanol, *n*-butanol, isobutanol, *tert*-butanol, acetone, acetonitrile, dichloromethane, and dimethyl sulfoxide. For example, the co-solvent system is selected from a co-solvent system comprising water and one organic solvent selected from the following: ethanol, isopropanol, and *tert-*butanol.

According to an embodiment of the present disclosure, in the co-solvent system of water and an organic solvent, the volume percentage of the organic solvent ranges from 1% to 99%, for example, 10% to 90%, preferably 50% to 90%, and an example thereof may be 50%, 55%, 60%, 65%, 70%, 75%, 80%, 85%, 90%, or 95%.

For example, the co-solvent system of water and an organic solvent is selected from a co-solvent system of water and *tert*-butanol. In the co-solvent system, the volume percentage of *tert*-butanol may range from 50% to 95%, preferably 60% to 90%, and more preferably 65% to 85%, and an example thereof is 65%, 70%, 75%, 80%, or 85%.

According to an embodiment of the present disclosure, when the active pharmaceutical ingredient has a solubility in water lower than that in an organic solvent, the solvent is selected from a co-solvent system comprising water and said organic solvent.

According to an embodiment of the present disclosure, the mass-to-volume ratio of the active pharmaceutical ingredient to the solvent is ≥ 1 mg/mL, preferably ≥ 5 mg/mL, for example, ≥ 10 mg/mL. According to an embodiment of the present disclosure, the solution comprising the active pharmaceutical ingredient optionally further comprises:
1) a pharmaceutically acceptable stabilizer.

According to an embodiment of the present disclosure, the pharmaceutically acceptable stabilizer is selected from one or more of a long-chain alcohol (e.g., a C₁₈-C₃₀ long-chain alcohol) and a C₁₈-C₃₀ acid or a salt thereof.

According to an embodiment of the present disclosure, the pharmaceutically acceptable stabilizer is selected from one or more of cholesterol, octadecanol, cholic acid or a salt thereof, deoxycholic acid or a salt thereof, taurodeoxycholic acid or a salt thereof, ursodeoxycholic acid or a salt thereof, chenodeoxycholic acid or a salt thereof, and glycocholic acid or a salt thereof.

According to an embodiment of the present disclosure, the pharmaceutically acceptable stabilizer is selected from one or more of cholesterol, octadecanol, cholic acid, deoxycholic acid, ursodeoxycholic acid, chenodeoxycholic acid, glycocholic acid, sodium cholate, sodium deoxycholate, and sodium taurodeoxycholate.

According to an embodiment of the present disclosure, the molar ratio of the active pharmaceutical ingredient to the stabilizer ranges from 0.5:1 to 30:1, preferably 1:1 to 15:1, preferably 1:1 to 10:1, and more preferably 1:1 to 7:1, and more preferably is 1:1, 1.5:1, 2:1, 2.5:1, 3:1, 3.5:1, 4:1, 4.5:1, 5:1, 5.5:1, 6:1, 6.5:1, or 7: 1.

According to an embodiment of the present disclosure, the solution comprising the active pharmaceutical ingredient optionally further comprises:
2) a pharmaceutically acceptable dispersant.

According to an embodiment of the present disclosure, the pharmaceutically acceptable dispersant is selected from one or more of a polysorbate (Tween), poloxamer, polyoxyethylene fatty acid ester, polyoxyethylene fatty acid ether, sucrose fatty acid ester, sodium dodecyl sulfate, macrogol 15 hydroxystearate (HS-15), and a phospholipid.

According to an embodiment of the present disclosure, the polysorbate is selected from one or more of polysorbate 80 (Tween 80) and polysorbate 20 (Tween 20).

According to an embodiment of the present disclosure, the phospholipid is selected from one or more of DMPC (dimyristoylphosphatidylcholine), DPPC (dipalmitoylphosphatidylcholine), DLPC (dilauroylphosphatidylcholine), SPC (soybean phosphatidylcholine), EPC (egg phosphatidylcholine), DEPC (dierucoylphosphatidylcholine), DOPC (dioleoylphosphatidylcholine), and POPC (palmitoyloleoylphosphatidylcholine).

According to an embodiment of the present disclosure, the molar ratio of the active pharmaceutical ingredient to the pharmaceutically acceptable dispersant ranges from 50:1 to 1:1, preferably 40:1 to 2:1, more preferably 30:1 to 2:1, and more preferably 20:1 to 2:1, and more preferably is 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, or 2:1.

According to an embodiment of the present disclosure, the solution comprising the active pharmaceutical ingredient optionally further comprises:
3) a pharmaceutically acceptable inorganic salt.

According to an embodiment of the present disclosure, the pharmaceutically acceptable inorganic salt has a solubility in water at 20 °C of greater than 10 g/100 mL.

According to an embodiment of the present disclosure, the inorganic salt is selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride.

According to an embodiment of the present disclosure, the molar ratio of the active pharmaceutical ingredient to the pharmaceutically acceptable inorganic salt is ≥ 1:0.02, preferably ranges from 1:0.02 to 1:2.0, and more preferably is 1:0.02, 1:0.05, 1:0.1, 1:0.2, 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, or 1:2.0.

According to an embodiment of the present disclosure, the solution comprising the active pharmaceutical ingredient optionally further comprises:
4) a pharmaceutically acceptable pH regulator.

According to an embodiment of the present disclosure, the pH regulator is selected from an alkaline pH regulator and an acidic pH regulator.

According to an embodiment of the present disclosure, the alkaline pH regulator is selected from one or more of meglumine, tromethamine, sodium hydroxide, potassium hydroxide, sodium carbonate, sodium bicarbonate, magnesium hydroxide, calcium hydroxide, sodium phosphate, disodium hydrogen phosphate, potassium phosphate, dipotassium hydrogen phosphate, sodium citrate, sodium acetate, potassium acetate, ammonium acetate, triethylamine, ethanolamine, and diethanolamine, preferably one or more of sodium hydroxide, potassium hydroxide, magnesium hydroxide, sodium bicarbonate, and calcium hydroxide.

According to an embodiment of the present disclosure, the acidic pH regulator is selected from one or more of citric acid, tartaric acid, maleic acid, malic acid, fumaric acid, succinic acid, hydrochloric acid, phosphoric acid, ascorbic acid, lactic acid, acetic acid, methanesulfonic acid, oxalic acid, sulfuric acid, glycine, sodium dihydrogen phosphate, and potassium dihydrogen phosphate, preferably one or more of citric acid, tartaric acid, maleic acid, malic acid, fumaric acid, succinic acid, hydrochloric acid, phosphoric acid, lactic acid, and acetic acid.

According to an embodiment of the present disclosure, the solution comprising the active pharmaceutical ingredient optionally further comprises:
5) a pharmaceutically acceptable osmotic pressure regulator.

According to an embodiment of the present disclosure, the osmotic pressure regulator is selected from one or more of sodium chloride, mannitol, lactose, glucose, sorbitol, and glycerol, preferably one or more of sodium chloride, glucose, sorbitol, and mannitol.

According to an embodiment of the present disclosure, the osmotic pressure of the suspension injection is comparable to the physiological osmotic pressure.

According to an embodiment of the present disclosure, the solution comprising the active pharmaceutical ingredient optionally further comprises:
6) a pharmaceutically acceptable lyoprotectant.

According to an embodiment of the present disclosure, the pharmaceutically acceptable lyoprotectant is selected from one or more of mannitol, lactose, trehalose, sucrose, maltose, dextrin, stachyose, gelatin, pectin, sugar alcohol, sorbitol, proline, histidine, lysine, arginine, polyethylene glycol, and polyvinylpyrrolidone, preferably one or more of mannitol, lactose, trehalose, sucrose, and polyvinylpyrrolidone.

According to an embodiment of the present disclosure, the solid pharmaceutical composition optionally does not comprise a pharmaceutically acceptable suspending agent, wherein the suspending agent is preferably a water-soluble polymer compound, including but not limited to one or more of carboxymethyl cellulose or a sodium salt thereof, hydroxypropyl cellulose or a sodium salt thereof, hydroxypropyl methylcellulose or a sodium salt thereof, methyl cellulose or a sodium salt thereof, hydroxyethyl cellulose or a sodium salt thereof, sodium hyaluronate, and polyvinylpyrrolidone.

According to an embodiment of the present disclosure, the preparation method comprises: completely dissolving all components in the solution to obtain a solution, and drying to obtain the solid composition.

According to an embodiment of the present disclosure, the preparation method comprises: completely dissolving all components in the solution to obtain a solution, and sterilizing, filtering, and drying to obtain the solid composition.

According to an embodiment of the present disclosure, the active pharmaceutical ingredient may be selected from ropivacaine, a pharmaceutically acceptable salt of ropivacaine, and other active pharmaceutical ingredients that can be used for diseases or disorders.

According to an embodiment of the present disclosure, the solid pharmaceutical composition may be selected from a solid composition of ropivacaine or a pharmaceutically acceptable salt thereof, which may include, but is not limited to, the solid composition of ropivacaine or the pharmaceutically acceptable salt thereof described in the context of this specification.

According to an embodiment of the present disclosure, the pharmaceutically acceptable salt of ropivacaine is an acidic salt of ropivacaine, i.e., an addition salt of ropivacaine with a pharmaceutically acceptable acid. For example, the acidic salt of ropivacaine includes, but is not limited to, one or more selected from the following salts of ropivacaine: acetate, benzoate, benzenesulfonate, hydrobromide, camphorsulfonate, hydrochloride, citrate, edisylate, fumarate, glucoheptonate, gluconate, glucuronate, hydroiodide, isethionate, lactate, lactobionate, dodecylsulfate, malate, maleate, methanesulfonate, naphthoate, naphthalenesulfonate, nitrate, stearate, palmitate, myristate, laurate, oleate, oxalate, pamoate, phosphate, polygalacturonate, succinate, sulfate, sulfosalicylate, tartrate, toluenesulfonate, and trifluoroacetate.

According to an embodiment of the present disclosure, when the active pharmaceutical ingredient in the solution is a pharmaceutically acceptable salt, for example, a pharmaceutically acceptable acid addition salt or base addition salt, it may react with the pH regulator in the solution, thereby preparing *in situ* a corresponding free active ingredient, such as a free basic active ingredient or a free acidic active ingredient.

Alternatively, when the active pharmaceutical ingredient in the solution is a free basic active ingredient or a free acidic active ingredient, it may react with the pH regulator in the solution, thereby preparing *in situ* a corresponding pharmaceutically acceptable salt.

For example, when the active pharmaceutical ingredient in the solution is a pharmaceutically acceptable salt of ropivacaine, ropivacaine can be prepared *in situ* by reaction of the pharmaceutically acceptable salt of ropivacaine with the alkaline pH regulator.

According to an embodiment of the present disclosure, the pharmaceutically acceptable salt of ropivacaine has the definition described above.

According to an embodiment of the present disclosure, when the free active ingredient or the pharmaceutically acceptable salt is prepared *in situ* as described above, the molar ratio of the active pharmaceutical ingredient used as a reaction starting material to the pH regulator may be determined by a person skilled in the art, for example, as 3:1-1:3. For example, when the active pharmaceutical ingredient used as the reaction starting material is a pharmaceutically acceptable salt of ropivacaine, the molar ratio of the active pharmaceutical ingredient to the alkaline pH regulator may range from 1:0.5 to 1:2.0, preferably 1:0.8 to 1:1.5, and preferably 1: 1.0 to 1:1.2. As an example, the molar ratio of the pharmaceutically acceptable salt of ropivacaine to the alkaline pH regulator is 1:0.6, 1:0.7, 1:0.8, 1:0.9, 1:1.0, 1:1.1, 1:1.2, 1:1.3, 1:1.4, 1:1.5, 1:1.6, 1:1.7, 1:1.8, 1:1.9, or 1:2.0.

The present disclosure further provides a solid pharmaceutical composition prepared by the preparation method described above, such as a solid composition of ropivacaine or a pharmaceutically acceptable salt thereof.

The present disclosure further provides a preparation method for a suspension, the preparation method comprising reconstituting the solid pharmaceutical composition described above, thereby obtaining the suspension.

According to an embodiment of the present disclosure, the preparation method comprises mixing the solid pharmaceutical composition described above with a clinically acceptable diluent, thereby obtaining the suspension.

According to an embodiment of the present disclosure, the clinically acceptable diluent includes, but is not limited to, one or more of water for injection, a sodium chloride injection, a glucose injection, a Ringer's lactate solution, a sucrose solution, a xylitol solution, and a pH buffer.

According to an embodiment of the present disclosure, the sodium chloride injection includes, but is not limited to, a 0.9% sodium chloride injection.

According to an embodiment of the present disclosure, the glucose injection includes, but is not limited to, a 5% glucose injection.

According to an embodiment of the present disclosure, the pH buffer is selected from a buffer with a pH ranging from 5.0 to 7.4, for example, including but not limited to one or more of a phosphate buffer, a glycine buffer, a histidine buffer, a citrate buffer, and an acetate buffer.

According to an embodiment of the present disclosure, the pH of the suspension ranges from 6.0 to 9.0, preferably 6.0 to 8.5, preferably 6.0 to 8.0, and more preferably 6.0 to 7.5, and more preferably is 6.2, 6.4, 6.6, 6.8, 7.0, 7.2, or 7.4.

According to an embodiment of the present disclosure, in the suspension, the concentration of the active pharmaceutical ingredient ranges from about 0.1 mg/mL to about 300 mg/mL, preferably about 1 mg/mL to about 50 mg/mL, and more preferably about 2.5 mg/mL to about 40 mg/mL. As an example, in the suspension, the concentration of ropivacaine or the pharmaceutically acceptable salt thereof is about 0.1 mg/mL, 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, or 100 mg/mL.

According to an embodiment of the present disclosure, the suspension is a suspension of ropivacaine or a pharmaceutically acceptable salt thereof, which includes, but is not limited to, the suspension of ropivacaine or the pharmaceutically acceptable salt thereof described above in the context of this specification.

The present disclosure further provides a suspension prepared by the preparation method described above, such as a suspension of ropivacaine or a pharmaceutically acceptable salt thereof.

The present disclosure further provides use of a pharmaceutically acceptable inorganic salt and/or a pharmaceutically acceptable stabilizer as a stabilizer for particles (e.g., crystals) of ropivacaine or a pharmaceutically acceptable salt thereof.

The present disclosure further provides use of a pharmaceutically acceptable inorganic salt and/or a pharmaceutically acceptable stabilizer as a stabilizer for particles of ropivacaine or a pharmaceutically acceptable salt thereof (e.g., crystals) in the preparation method for the suspension of ropivacaine or the pharmaceutically acceptable salt thereof, or the solid composition of ropivacaine or the pharmaceutically acceptable salt thereof described above.

According to an embodiment of the present disclosure, the pharmaceutically acceptable inorganic salt and/or the pharmaceutically acceptable stabilizer is used for promoting the (stable) presence of ropivacaine or the pharmaceutically acceptable salt thereof in the form of particles (e.g., crystals), preferably in the form of regular crystals, such as rod-shaped or block-shaped crystals, in the suspension or the solid composition.

According to an embodiment of the present disclosure, the inorganic salt is selected from a pharmaceutically acceptable inorganic salt having a solubility in water of greater than 10 g/100 mL of water at 20 °C.

According to an embodiment of the present disclosure, the inorganic salt is selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride.

According to an embodiment of the present disclosure, the pharmaceutically acceptable stabilizer is selected from a C₁₈-C₃₀ long-chain alcohol and a C₁₈-C₃₀ acid or a salt thereof.

According to an embodiment of the present disclosure, the pharmaceutically acceptable stabilizer is selected from one or more of cholesterol, octadecanol, cholic acid, deoxycholic acid, ursodeoxycholic acid, chenodeoxycholic acid, glycocholic acid, sodium cholate, sodium deoxycholate, and sodium taurodeoxycholate.

The present disclosure also provides use of the suspension or the solid composition described above in the preparation of a medicament for producing anesthesia or relieving pain.

The present disclosure further provides use of the suspension or the solid composition described above in the preparation of a medicament for treating and/or preventing pain.

The present disclosure further provides the suspension or the solid composition described above for use in treating and/or preventing pain.

According to an embodiment of the present disclosure, the suspension or the solid composition is administered around a nerve or to a surgical wound.

According to an embodiment of the present disclosure, the pain is chronic postoperative pain or labor pain.

The present disclosure further provides a method for producing anesthesia, the method comprising administering to an individual the suspension or the solid composition described above.

The present disclosure further provides a method for treating, ameliorating, and/or preventing pain, the method comprising administering to an individual the suspension or the solid composition described above.

According to an embodiment of the present disclosure, the administration is parenteral administration and/or topical administration.

According to an embodiment of the present disclosure, the parenteral administration is selected from intravenous administration, intramuscular administration, subcutaneous injection, intraarticular injection, epidural administration, subarachnoid administration, incision perfusion administration, infiltration, and nerve block, preferably intramuscular administration, subcutaneous injection, intraarticular injection, epidural administration, subarachnoid administration, incision perfusion administration, infiltration, and nerve block.

As used herein, the term "individual" includes mammals and humans.

According to an embodiment of the present disclosure, the suspension or the solid composition described above provides a sustained release of the drug in the individual for no less than 12 h. In some embodiments, a sustained release of the drug for no less than 24 h is provided. In some embodiments, a sustained release of the drug for no less than 36 h is provided. In some embodiments, a sustained release of the drug for no less than 48 h is provided. In some embodiments, a sustained release of the drug for no less than 60 h is provided. In some embodiments, a sustained release of the drug for no less than 72 h is provided. In some embodiments, a sustained release of the drug for no less than 120 h is provided. In some embodiments, a sustained release of the drug for no less than 168 h is provided.

According to an embodiment of the present disclosure, the suspension or the solid composition described above can achieve a peak drug concentration within about 3 h, for example, within about 2 h, preferably within about 1.5 h, more preferably within about 1 h, and most preferably within about 30 min, after administration to the individual.

### Beneficial Effects

The present disclosure provides a novel solid composition of ropivacaine or a pharmaceutically acceptable salt thereof, wherein the solid composition formulation is a freeze-dried powder for injection, which forms a suspension upon reconstitution; in the solid composition and the suspension, ropivacaine or the pharmaceutically acceptable salt thereof is stably present in the form of particles (e.g., crystals) in the system.

Adding an acceptable inorganic salt and/or a pharmaceutically acceptable stabilizer to the formulation system described above can significantly improve the crystal morphology of ropivacaine or the pharmaceutically acceptable salt thereof in the suspension or the solid composition, preferably forming regular crystals, such as rod-shaped or block-shaped crystals, of the ropivacaine or the pharmaceutically acceptable salt thereof; after administration, the drug crystals are slowly dissolved, thereby achieving a sustained-release effect.

The suspension (e.g., suspension injection) and the solid composition of the present disclosure feature good stability, controllable product quality, ease of administration, minor irritation, and favorable medication safety and tolerability.

The sustained-release formulation system provided by the present disclosure enables the active pharmaceutical ingredient to achieve favorable release performance and reduces the possibility of burst release. It is particularly suitable for developing pharmaceutical formulations with anesthetic and analgesic activities, offering more advantages over other prior art, such as simple preparation process, guaranteed sterility level through sterilization and filtration, and ease of scale-up production. It enables the release of the analgesic active ingredient to be sustained and stable, with good tolerability and few side effects in patients.

### Terms and Abbreviations

Unless otherwise stated, all numbers indicating contents, concentrations, ratios, weights, percentages, technical effects, etc., used in this specification and claims shall, in all cases, be understood as being modified by the term "about" or "approximately". "About" represents a range of ±10% of the numerical value modified thereby.

Accordingly, unless indicated to the contrary, the numerical parameters set forth in the following specification and appended claims are approximations. Unless otherwise stated, the terms used herein have the meanings that are commonly understood by those skilled in the art. It should be understood by a person skilled in the art that the numerical parameters may vary depending on the desired properties and effects sought and obtained by the present disclosure, and each numerical parameter should be interpreted according to the number of significant digits and conventional rounding rules or as understood by a person skilled in the art.

Although the numerical ranges and parameters setting forth the broad scope of the present disclosure are approximations, the numerical values set forth in the specific examples are provided as precisely as possible. However, any numerical value inherently contains certain errors necessarily resulting from the standard deviation found in their respective tests or measurements. Each numerical range given in this specification will include each narrower numerical range that falls within this broader numerical range, as if such narrower numerical ranges were all explicitly written herein.

Unless otherwise stated, the definitions of excipients described in the specification and claims of the present application, including definitions thereof as examples, exemplary definitions, preferred definitions, definitions documented in tables, definitions of specific excipients provided in the examples, etc., may be arbitrarily combined and incorporated with each other. The excipients resulting from such combinations and incorporations shall fall within the scope of the present specification.

"D50" described in the context of this specification refers to the particle size at which the cumulative particle size distribution percentage of a sample reaches 50%. "D90" refers to the particle size at which the cumulative particle size distribution percentage of a sample reaches 90%.

The term "long-chain alcohol" means a higher alkanol, also known as a higher fatty alcohol, which includes saturated monohydric alcohols with twelve or more carbon atoms that are waxy solids. When "C₁₈-C₃₀ long-chain alcohol" is used in the context of this specification, it means a C₁₈-C₃₀ saturated monohydric alcohol, i.e., a saturated monohydric alcohol with 18 to 30 carbon atoms. As an example, the C₁₈-C₃₀ long-chain alcohol may be selected from cholesterol and octadecanol.

The term "particles" means solid geometric objects having a specific shape within a certain size range. Preferably, in the context of this specification, the particle size of "particles" is at the micron level. The term "regular crystals" means particles having a specific shape. The regular crystals in the context of this specification may be rod-shaped, spherical, block-shaped, etc.

The term "irregular crystals" means particles that do not have a specific shape.

The osmotic pressure of the pharmaceutical composition is comparable to the physiological osmotic pressure, and the osmotic pressure of the suspension injection is comparable to the physiological osmotic pressure; the meaning of "comparable to" is 300±50 mmol/L.

The term "freeze drying" comprises a pre-freezing procedure and a drying procedure. For example, the pre-freezing procedure comprises a cooling step 1, an annealing step, and a cooling step 2. Alternatively, for example, the drying procedure comprises a primary drying step and a secondary drying step.

The abbreviations used in this specification have the following definitions: TW80 for Tween 80; TW20 for Tween 20; HS-15 for macrogol 15 hydroxystearate; F68 for poloxamer 188; DMPC for dimyristoylphosphatidylcholine; DPPC for dipalmitoylphosphatidylcholine; DLPC for dilauroylphosphatidylcholine; SPC for soybean phosphatidylcholine; EPC for egg phosphatidylcholine; DEPC for dierucoylphosphatidylcholine; DOPC for dioleoylphosphatidylcholine; POPC for palmitoyloleoylphosphatidylcholine; HSPC for hydrogenated soybean phosphatidylcholine; DSPC for distearoylphosphatidylcholine; ROP for ropivacaine; CHO for cholesterol; TBA for *tert*-butanol; MgCl₂·6H₂O for magnesium chloride hexahydrate; and NaCl for sodium chloride.

### BRIEF DESCRIPTION OF THE DRAWINGS

FIG. 1 shows a microscopic morphology image of ropivacaine in example composition 6006;
FIG. 2 shows microscopic morphology images of ropivacaine in example compositions 6030 to 6035;
FIG. 3 shows microscopic morphology images of ropivacaine in example compositions 6036, 6037, 6039, 6040, 6041, 6042, 6044, and 6045;
FIG. 4 shows microscopic morphology images of ropivacaine in example compositions 6046 and 6049;
FIG. 5 shows microscopic morphology images of ropivacaine in example compositions 6010 and 6064;
FIG. 6 shows microscopic morphology images of ropivacaine in example compositions 6074, 6076, and 6078;
FIG. 7 shows microscopic morphology images of ropivacaine in example compositions 6083 and 6087;
FIG. 8 shows microscopic morphology images of ropivacaine in example compositions 6091, 6097, 6098, and 6104;
FIG. 9 shows microscopic morphology images of ropivacaine in example composition 6105 after reconstitution with purified water, an aqueous sodium chloride solution, and an aqueous magnesium chloride solution;
FIG. 10 shows microscopic morphology images of ropivacaine in example compositions 6106 and 6107;
FIG. 11 shows an electron microscopic morphology image of ropivacaine in example composition 6106;
FIG. 12 shows microscopic morphology images of ropivacaine in example compositions 6106, 6126, 6127, and 6128;
FIG. 13 shows a crystal morphology image of ropivacaine in example composition 6129;
FIG. 14 shows *in-vivo* plasma concentration curves in rats for example compositions 6106 and 6143;
FIG. 15 shows an efficacy comparison in rats between example composition 6144 and EXPAREL; and
FIG. 16 shows a local safety comparison between example composition 6154 and Comparative Example 3.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented based on the content described above in the present disclosure are encompassed within the claimed scope of the present disclosure.

Unless otherwise stated, the starting materials and reagents used in the following examples are all commercially available products or can be prepared using known methods.

Unless otherwise stated, the percentage content of *tert*-butanol (TBA) in the aqueous *tert*-butanol solution in the following examples is expressed as a volume percentage content. When a percentage below 100% is described together with TBA (including but not limited to 80% TBA, 75% TBA, etc.), it means an aqueous *tert*-butanol solution containing *tert*-butanol at the stated percentage. When a percentage of 100% is described together with TBA (i.e., 100% TBA), it means *tert*-butanol without additional addition of water.

Sources of experimental animals:
Beagle dogs: Jiangsu Marshall Biotechnology Co., Ltd.; male.
Rats: Zhejiang Vital River Laboratory Animal Technology Co., Ltd., and Nantong University Laboratory Animal Center; male.

### Example 1

### Investigation of Conventional Freeze-Dried Compositions

### (1) Compositions containing lyoprotectants

The formula amounts of ropivacaine and a lyoprotectant were weighed according to Table 1-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The reconstitution dispersibility was then observed under a microscope (Olympus, CX41).

**Table 1-1. Compositions containing lyoprotectants**

| Composition No. | 6001 | 6002 | 6003 |
|---|---|---|---|
| | mg | mg | mg |
| ROP | 20 | 20 | 20 |
| Mannitol | 10 | / | / |
| Glycine | / | 10 | / |
| Trehalose | / | / | 10 |
| 80%TBA | Added to make up to 2 mL | Added to make up to 2 mL | Added to make up to 2 mL |

**Table 1-2. Reconstitution dispersibility results**

| Composition No. | Reconstitution dispersibility |
|---|---|
| 6001 | Could not be dispersed |
| 6002 | Could not be dispersed |
| 6003 | Could not be dispersed |

**Table 1-3. Freeze drying method**

| Freeze drying stage | Temperature/°C | Heating/cooling time/min | Duration/min | Vacuum degree/Pa |
|---|---|---|---|---|
| | Setting value | Setting value | Setting value | |
| Pre-freezing-cooling | -30 | 120 | 60 | / |
| Pre-freezing-annealing | -2 | 30 | 600 | / |
| Pre-freezing-cooling | -45 | 180 | 120 | / |
| Primary drying Stage 1 | -30 | 30 | 180 | 10~30 |
| Primary drying Stage 2 | 0 | 30 | 360 | 10~50 |
| Secondary drying Stage 1 | 30 | 30 | 180 | 10~30 |
| Secondary drying Stage 2 | 40 | 30 | 180 | 0~10 |

The results showed that: the compositions containing only the lyoprotectants could not be dispersed after reconstitution.

### (2) Compositions containing suspending agents

The formula amounts of ropivacaine and a suspending agent were weighed according to Table 1-4 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The reconstitution dispersibility was then observed under a microscope (Olympus, CX41).

**Table 1-4. Compositions containing suspending agents**

| Composition No. | 6004 | 6005 |
|---|---|---|
| | mg | mg |
| ROP | 100 | 100 |
| Polyethylene glycol 4000 | 50 | / |
| Povidone K30 | / | 50 |
| 80%TBA | Added to make up to 10 mL | Added to make up to 10 mL |

**Table 1-5. Reconstitution dispersibility results**

| Composition No. | Solution stability before freeze drying | Reconstitution dispersibility |
|---|---|---|
| 6004 | Clear solution | Could not be dispersed |
| 6005 | Clear solution | Could not be dispersed |

The results showed that: the compositions containing only commonly used suspending agents also could not be dispersed after reconstitution.

### Example 2

### Study on Dispersants

### (1) Compositions containing ordinary dispersants

The formula amounts of ropivacaine and a dispersant were weighed according to Table 2-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 2-1. Compositions containing ordinary dispersants**

| Composition No. | 6006 | 6007 | 6008 | 6009 |
|---|---|---|---|---|
| | mg | mg | mg | mg |
| ROP | 40 | 40 | 40 | 40 |
| TW80 | 10 | 0 | 0 | 0 |
| TW20 | 0 | 10 | 0 | 0 |
| HS-15 | 0 | 0 | 10 | 0 |
| F68 | 0 | 0 | 0 | 10 |
| 75%TBA | Added to make up to 2 mL | Added to make up to 2 mL | Added to make up to 2 mL | Added to make up to 2 mL |

**Table 2-2. Reconstitution dispersibility and crystal morphology**

| Composition No. | Reconstitution dispersibility | Crystal morphology |
|---|---|---|
| 6006 | Easily dispersed | Irregular crystals |
| 6007 | Easily dispersed | Irregular crystals |
| 6008 | Easily dispersed | Irregular crystals |
| 6009 | Easily dispersed | Irregular crystals |

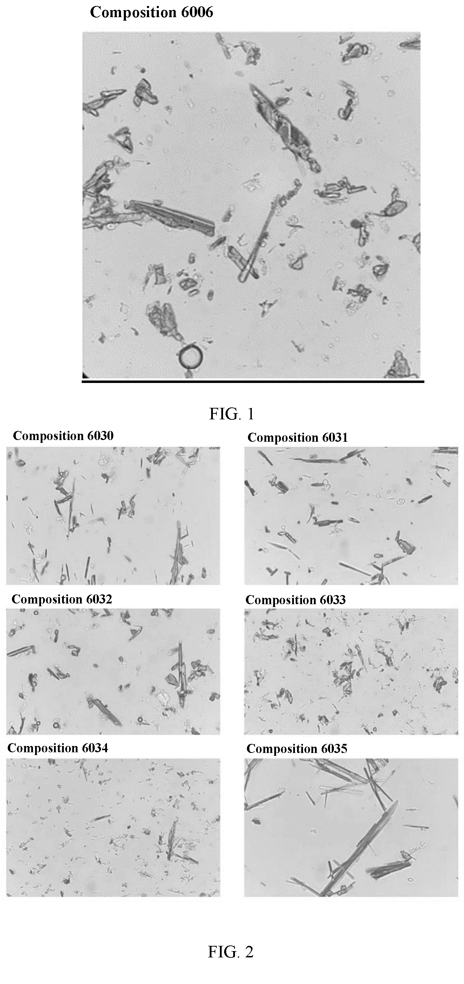

The results showed that: the addition of commonly used dispersants could improve the reconstitution performance of the compositions; however, microscopic observation revealed that the crystal morphology of ropivacaine in the suspensions after reconstitution was irregular; the crystal morphology of ropivacaine in composition 6006 after reconstitution is shown in FIG. 1, and the morphology of ropivacaine observed microscopically in other dispersant-containing compositions after reconstitution was similar to that in FIG. 1; furthermore, the crystals exhibited an aggregation phenomenon, with significant variation in crystal size.

### (2) Compositions containing phospholipid dispersants

The formula amounts of ropivacaine and a phospholipid dispersant were weighed according to Table 2-3 and added to a Schott bottle, and then an aqueous *tert*-butanol solution was added. The mixture was appropriately heated and stirred until complete dissolution, then filtered through a 0.22 µm filter membrane, aliquoted at 1.5 mL/vial, and subjected to freeze drying according to Table 1-3. 1.5 mL of normal saline was added to the freeze-dried powder, and the mixture was shaken until the powder was uniformly dispersed. The crystal morphology of ropivacaine after reconstitution was then observed under a microscope (Olympus, CX41). Table 2-3. Compositions containing phospholipid dispersants

**Table 2-3. Compositions containing phospholipid dispersants**

| Composition No. | 6010 | 6011 | 6012 | 6013 | 6014 | 6015 | 6016 | 6017 |
|---|---|---|---|---|---|---|---|---|
| | mg | mg | mg | mg | mg | mg | mg | mg |
| ROP | 400 | 400 | 400 | 400 | 400 | 400 | 400 | 400 |
| SPC | 100 | 0 | 0 | 0 | 0 | 0 | 0 | 0 |
| EPC | 0 | 100 | 0 | 0 | 0 | 0 | 0 | 0 |
| HSPC | 0 | 0 | 100 | 0 | 0 | 0 | 0 | 0 |
| DMPC | 0 | 0 | 0 | 100 | 0 | 0 | 0 | 0 |
| DPPC | 0 | 0 | 0 | 0 | 100 | 0 | 0 | 0 |
| DEPC | 0 | 0 | 0 | 0 | 0 | 100 | 0 | 0 |
| DOPC | 0 | 0 | 0 | 0 | 0 | 0 | 100 | 0 |
| DSPC | 0 | 0 | 0 | 0 | 0 | 0 | 0 | 100 |
| 75%TBA | Added to make up to 15 mL | Added to make up to 15 mL | Added to make up to 15 mL | Added to make up to 15 mL | Added to make up to 15 mL | Added to make up to 15 mL | Added to make up to 15 mL | Added to make up to 15 mL |

| | | | | | | | | |
|---|---|---|---|---|---|---|---|---|
| * 6012 and 6017 exhibited a crystallization phenomenon in the solution before freeze drying and thus were not further studied. | | | | | | | | |

**Table 2-4. Reconstitution dispersibility and crystal morphology**

| No. | Reconstitution dispersibility | Crystal morphology |
|---|---|---|
| 6010 | Easily dispersed | Irregular crystals |
| 6011 | Easily dispersed | Irregular crystals |
| 6013 | Easily dispersed | Irregular crystals |
| 6014 | Easily dispersed | Irregular crystals |
| 6015 | Easily dispersed | Irregular crystals |
| 6016 | Easily dispersed | Irregular crystals |

The results showed that: the addition of the phospholipid dispersants, except for HSPC and DSPC, could improve the reconstitution performance of the compositions; however, microscopic observation revealed that the crystal morphology of ropivacaine after reconstitution was irregular, similar to that in FIG. 1; furthermore, the crystals exhibited an aggregation phenomenon, with relatively significant variation in crystal size.

### (3) Study on effect of amount of dispersant on crystal morphology

Using polysorbate 20 and DMPC as representatives, the effects of different molar ratios of ropivacaine to dispersant on crystal morphology were investigated. The formula amounts of ropivacaine and a dispersant were weighed according to Tables 2-5 and 2-6 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 2-5. Compositions with different amounts of DMPC**

| Composition No. | 6018 | 6019 | 6020 | 6021 | 6022 | 6023 |
|---|---|---|---|---|---|---|
| | mg | mg | mg | mg | mg | mg |
| ROP:DMPC molar ratio | 50:1 | 30:1 | 10:1 | 5:1 | 2:1 | 1:1 |
| ROP | 40 | 40 | 40 | 40 | 40 | 40 |
| DMPC | 2 | 3.3 | 9.8 | 19.8 | 50 | 95 |
| 75%TBA | Added to make up to 2 mL | Added to make up to 2 mL | Added to make up to 2 mL | Added to make up to 2 mL | Added to make up to 2 mL | Added to make up to 2 mL |

**Table 2-6. Compositions with different amounts of polysorbate 20**

| Composition No. | 6024 | 6025 | 6026 | 6027 | 6028 | 6029 |
|---|---|---|---|---|---|---|
| | mg | mg | mg | mg | mg | mg |
| ROP:TW20 molar ratio | 50:1 | 30:1 | 10:1 | 5:1 | 2:1 | 1:1 |
| ROP | 40 | 40 | 40 | 40 | 40 | 40 |
| TW20 | 1.5 | 2.5 | 8 | 15 | 39 | 80 |
| 75%TBA | Added to make up to 2 mL | Added to make up to 2 mL | Added to make up to 2 mL | Added to make up to 2 mL | Added to make up to 2 mL | Added to make up to 2 mL |

The results showed that: when the molar ratio of ropivacaine to dispersant was in the range of 50:1 to 1:1, the reconstitution performance of the compositions could be improved; however, microscopic observation revealed that the crystal morphology of ropivacaine was irregular, similar to that in FIG. 1; furthermore, the crystals exhibited an aggregation phenomenon, with relatively significant variation in crystal size. The amount of the dispersant only affected the speed of dispersion, and did not affect the crystal morphology.

As can be seen from Example 2, with the addition of the dispersant, the crystals of ropivacaine in the suspension were irregular in morphology, non-uniform in dimension or size, and prone to aggregation. However, all these factors may lead to unstable and uncontrollable quality of the suspension product when used as an injection, and readily cause adverse consequences such as needle clogging during subsequent use of the injection. Therefore, the present disclosure aims to improve the crystal morphology and crystal size of ropivacaine in the suspension by screening and adding suitable additives and adjusting the proportions among the components, thereby controlling the product quality of the suspension.

### Example 3

### Study on Crystal Morphology Improvement

### (1) Study on solvent ratios

Using DMPC as a dispersant, the effects of different *tert*-butanol/water ratios on crystal morphology were investigated. The formula amounts of ropivacaine and DMPC were weighed according to Table 3-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 3-1. Formulation**

| Composition No. | 6030 | 6031 | 6032 | 6033 | 6034 | 6035 |
|---|---|---|---|---|---|---|
| | mg | mg | mg | mg | mg | mg |
| ROP:DMPC molar ratio | 5.6:1 | 5.6:1 | 5.6:1 | 5.6:1 | 5.6:1 | 5.6:1 |
| ROP | 200 | 200 | 200 | 200 | 200 | 200 |
| DMPC | 50 | 50 | 50 | 50 | 50 | 50 |
| TBA or an aqueous solution thereof | 65% TBA, added to make up to 10 mL | 70% TBA, added to make up to 10 mL | 75% TBA, added to make up to 10 mL | 80% TBA, added to make up to 10 mL | 90% TBA, added to make up to 10 mL | 100% TBA, added to make up to 10 mL |

**Table 3-2. Crystal morphology**

| Composition No. | Crystal morphology |
|---|---|
| 6030 | Irregular crystals |
| 6031 | Irregular crystals |
| 6032 | Irregular crystals |
| 6033 | Irregular crystals |
| 6034 | Irregular crystals |
| 6035 | Irregular crystals |

The results showed that: altering the proportion of *tert*-butanol on the basis of containing only the dispersant resulted in ropivacaine forming irregular crystals with no significant improvement in morphology. The crystal morphology of ropivacaine in compositions 6030 to 6035 is shown in FIG. 2.

### (2) Addition of alcohol or saccharide additives

By adding different alcohol additives on the basis of using DMPC and TW20 as dispersants, it was unexpectedly found that long-chain alcohol additives affected the crystal morphology of ropivacaine. The formula amounts of ropivacaine, a dispersant, and an additive were weighed according to Tables 3-3 and 3-4 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 3-3. Compositions containing water-soluble alcohol or saccharide additives**

| Composition No. | 6036 | 6037 | 6038 | 6039 | 6040 | 6041 | 6042 | 6043 | 6044 | 6045 |
|---|---|---|---|---|---|---|---|---|---|---|
| | mg | mg | mg | mg | mg | mg | mg | mg | mg | mg |
| ROP | 200 | 200 | 200 | 200 | 200 | 200 | 200 | 200 | 200 | 200 |
| DMPC | 50 | 50 | 50 | 50 | 50 | 0 | 0 | 0 | 0 | 0 |
| TW20 | 0 | 0 | 0 | 0 | 0 | 50 | 50 | 50 | 50 | 50 |
| Mannitol | 50 | 0 | 0 | 0 | 0 | 50 | 0 | 0 | 0 | 0 |
| Sorbitol | 0 | 50 | 0 | 0 | 0 | 0 | 50 | 0 | 0 | 0 |
| Lactose* | 0 | 0 | 50 | 0 | 0 | 0 | 0 | 50 | 0 | 0 |
| Sucrose | 0 | 0 | 0 | 50 | 0 | 0 | 0 | 0 | 50 | 0 |
| Trehalose | 0 | 0 | 0 | 0 | 50 | 0 | 0 | 0 | 0 | 50 |
| 75%TBA | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL |

| | | | | | | | | | | |
|---|---|---|---|---|---|---|---|---|---|---|
| * The lactose-containing compositions 6038 and 6043 failed to achieve complete dissolution and thus were not further studied.- Compositions containing water-insoluble alcohol additives | | | | | | | | | | |

**Table 3-4. Compositions containing water-insoluble alcohol additives**

| Composition No. | 6046 | 6047 | 6048 | 6049 | 6050 | 6051 | 6052 | 6053 |
|---|---|---|---|---|---|---|---|---|
| | mg | mg | mg | mg | mg | mg | mg | mg |
| ROP | 200 | 200 | 200 | 200 | 200 | 200 | 200 | 200 |
| DMPC | 50 | 50 | 50 | 50 | 0 | 0 | 0 | 0 |
| TW20 | 0 | 0 | 0 | 0 | 50 | 50 | 50 | 50 |
| CHO | 50 | 0 | 0 | 0 | 50 | 0 | 0 | 0 |
| Tetradecanol | 0 | 50 | 0 | 0 | 0 | 50 | 0 | 0 |
| Hexadecanol | 0 | 0 | 50 | 0 | 0 | 0 | 50 | 0 |
| Octadecanol | 0 | 0 | 0 | 50 | 0 | 0 | 0 | 50 |
| 75%TBA | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL |

**Table 3-5. Crystal morphology results**

| Composition No. | Crystal morphology | Composition No. | Crystal morphology |
|---|---|---|---|
| 6036 | Irregular crystals | 6046 | **Regular crystals (rod-shaped)** |
| 6037 | Irregular crystals | 6047 | Irregular crystals |
| 6039 | Irregular crystals | 6048 | Irregular crystals |
| 6040 | Irregular crystals | 6049 | **Regular crystals (rod-shaped)** |
| 6041 | Irregular crystals | 6050 | **Regular crystals (rod-shaped)** |
| 6042 | Irregular crystals | 6051 | Irregular crystals |
| 6044 | Irregular crystals | 6052 | Irregular crystals |
| 6045 | Irregular crystals | 6053 | **Regular crystals (rod-shaped or block-shaped)** |

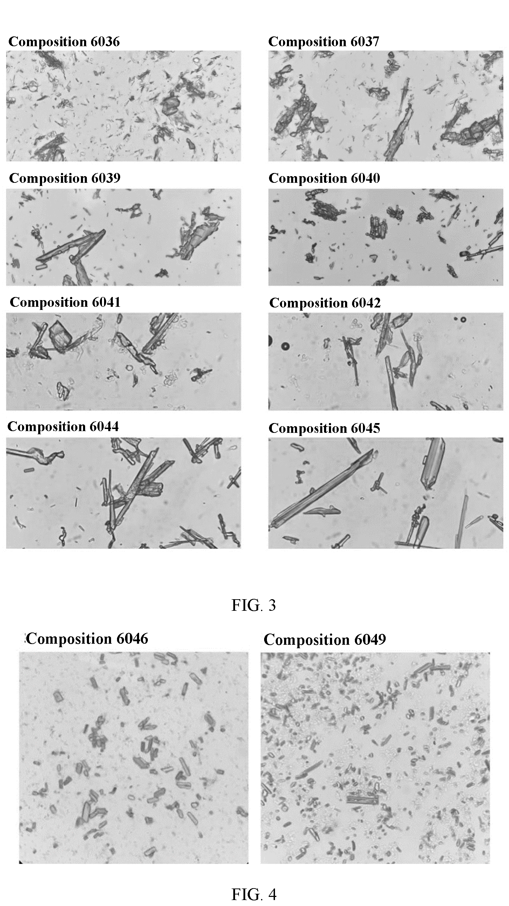

The results showed that: according to the alcohol additive screening results, ropivacaine was found to form irregular crystals after reconstitution of the compositions containing water-soluble alcohol or saccharide additives, as shown in FIG. 3; it was unexpectedly found that among the water-insoluble alcohol additives, only the addition of cholesterol and octadecanol could improve the crystal morphology, making the crystals regular rod-shaped or block-shaped, while the other alcohol additives all resulted in irregular crystals (similar to those in FIG. 3). FIG. 4 shows crystal morphology images of ropivacaine in compositions 6046 and 6049.

Taking cholesterol as an example, the crystal morphology of ropivacaine in different dispersants was investigated. The formula amounts of ropivacaine, a dispersant, and an additive were weighed according to Tables 3-6 and 3-7 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41), and the results were all similar to those in FIG. 4. The D50 and D90 were measured using a laser particle size analyzer (OMEC, Topsizer).

**Table 3-6. Compositions containing ordinary dispersants**

| Composition No. | 6054 | 6055 | 6056 |
|---|---|---|---|
| | mg | mg | mg |
| ROP | 200 | 200 | 200 |
| TW80 | 50 | 0 | 0 |
| TW20 | 0 | 50 | 0 |
| HS15 | 0 | 0 | 50 |
| CHO | 50 | 50 | 50 |
| 75%TBA | Added to make up to 10 mL | Added to make up to 10 mL | Added to make up to 10 mL |
| D50, µm | 12.0 | 13.6 | 12.9 |
| D90, µm | 39.4 | 41.5 | 38.3 |

**Table 3-7. Compositions containing phospholipid dispersants**

| Composition No. | 6058 | 6059 | 6060 | 6061 | 6062 | 6063 |
|---|---|---|---|---|---|---|
| | mg | mg | mg | mg | mg | mg |
| ROP | 200 | 200 | 200 | 200 | 200 | 200 |
| SPC | 50 | 0 | 0 | 0 | 0 | 0 |
| EPC | 0 | 50 | 0 | 0 | 0 | 0 |
| DMPC | 0 | 0 | 50 | 0 | 0 | 0 |
| DPPC | 0 | 0 | 0 | 50 | 0 | 0 |
| DEPC | 0 | 0 | 0 | 0 | 50 | 0 |
| DOPC | 0 | 0 | 0 | 0 | 0 | 50 |
| CHO | 50 | 50 | 50 | 50 | 50 | 50 |
| 75%TBA | Added to make up to 10 mL | Added to make up to 10 mL | Added to make up to 10 mL | Added to make up to 10 mL | Added to make up to 10 mL | Added to make up to 10 mL |
| D50, µm | 9.9 | 14.0 | 9.2 | 12.9 | 13.4 | 10.6 |
| D90, µm | 32.8 | 41.4 | 27.7 | 31.8 | 35.6 | 32.1 |

### (3) Study on cholesterol analogues

Substances structurally analogous to cholesterol were selected for investigation. The formula amounts of ropivacaine, a dispersant, and an additive were weighed according to Tables 3-8 and 3-9 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 3-8. Information on compositions**

| Composition No. | 6174 | 6175 | 6176 | 6177 | 6178 |
|---|---|---|---|---|---|
| | mg | mg | mg | mg | mg |
| ROP | 200 | 200 | 200 | 200 | 200 |
| DMPC | 50 | 50 | 50 | 50 | 50 |
| Cholic acid | 50 | 0 | 0 | 0 | 0 |
| Ursodeoxycholic acid | 0 | 50 | 0 | 0 | 0 |
| Deoxycholic acid | 0 | 0 | 50 | 0 | 0 |
| Chenodeoxycholic acid | 0 | 0 | 0 | 50 | 0 |
| Glycocholic acid | 0 | 0 | 0 | 0 | 60 |
| 80%TBA | Added to make up to 10 mL | Added to make up to 10 mL | Added to make up to 10 mL | Added to make up to 10 mL | Added to make up to 10 mL |

**Table 3-9. Information on compositions**

| Composition No. | 6179 | 6180 | 6181 |
|---|---|---|---|
| | mg | mg | mg |
| ROP | 200 | 200 | 200 |
| DMPC | 50 | 50 | 50 |
| Sodium deoxycholate | 159 | 0 | 0 |
| Sodium cholate | 0 | 165 | 0 |
| Sodium taurodeoxycholate hydrate | 0 | 0 | 200 |
| 80%TBA | Added to make up to 10 mL | Added to make up to 10 mL | Added to make up to 10 mL |

**Table 3-10. Crystal morphology results**

| Composition No. | Crystal morphology | Composition No. | Crystal morphology |
|---|---|---|---|
| 6174 | **Regular crystals (rod-shaped)** | 6178 | **Regular crystals (rod-shaped)** |
| 6175 | **Regular crystals (rod-shaped)** | 6179 | **Regular crystals (rod-shaped)** |
| 6176 | **Regular crystals (rod-shaped)** | 6180 | **Regular crystals (rod-shaped)** |
| 6177 | **Regular crystals (rod-shaped)** | 6181 | **Regular crystals (rod-shaped)** |

The results showed that: in the present disclosure, it was unexpectedly found that cholic acid and derivatives or salts thereof could improve the crystal morphology, making the crystals regular rod-shaped or block-shaped.

### (4) Addition of metal ion additives

On the basis of dispersants, metal ion additives were added to investigate their effects on the crystal morphology of ropivacaine. The formula amounts of ropivacaine, a dispersant, and an additive were weighed according to Tables 3-11 and 3-12 and added to a Schott bottle, and then an aqueous *tert-*butanol solution was added. The mixture was appropriately heated and stirred until complete dissolution, then filtered through a 0.22 µm filter membrane, aliquoted at 1.5 mL/vial or 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41).

**Table 3-11. Compositions containing metal ions**

| Composition No. | 6064 | 6065 | 6066 | 6067 | 6068 | 6069 |
|---|---|---|---|---|---|---|
| | mg | mg | mg | mg | mg | mg |
| ROP | 400 | 400 | 400 | 400 | 400 | 400 |
| SPC | 100 | 0 | 0 | 0 | 0 | 0 |
| EPC | 0 | 100 | 0 | 0 | 0 | 0 |
| DMPC | 0 | 0 | 100 | 0 | 0 | 0 |
| DPPC | 0 | 0 | 0 | 100 | 0 | 0 |
| DEPC | 0 | 0 | 0 | 0 | 100 | 0 |
| DOPC | 0 | 0 | 0 | 0 | 0 | 100 |
| Magnesium chloride | 406 | 406 | 406 | 406 | 406 | 406 |
| 75%TBA | Added to make up to 15 mL | Added to make up to 15 mL | Added to make up to 15 mL | Added to make up to 15 mL | Added to make up to 15 mL | Added to make up to 15 mL |

**Table 3-12. Compositions containing metal ions**

| Composition No. | 6070 | 6071 | 6072 | 6073 |
|---|---|---|---|---|
| | mg | mg | mg | mg |
| ROP | 200 | 200 | 200 | 200 |
| TW80 | 50 | 0 | 0 | 0 |
| TW20 | 0 | 50 | 0 | 0 |
| HS15 | 0 | 0 | 50 | 0 |
| F68 | 0 | 0 | 0 | 50 |
| Magnesium chloride | 202.8 | 202.8 | 202.8 | 202.8 |
| 75%TBA | Added to make up to 10 mL | Added to make up to 10 mL | Added to make up to 10 mL | Added to make up to 10 mL |

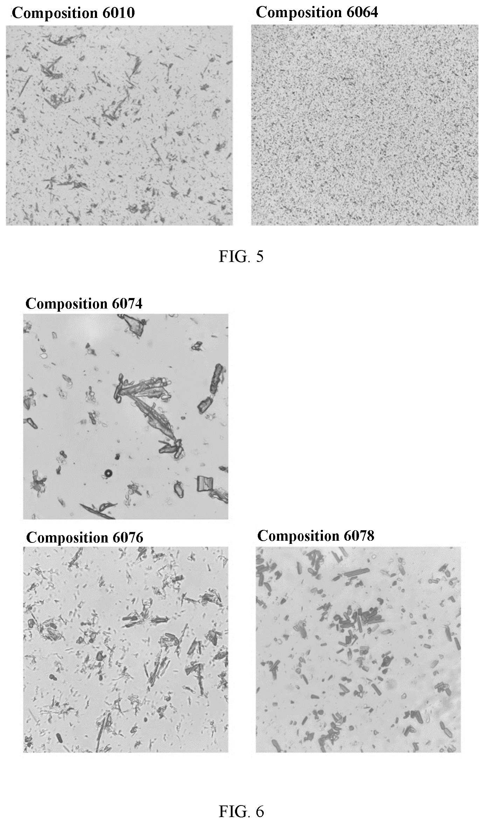

The results showed that: in the present disclosure, it is unexpectedly found that the addition of metal ions to the system could reduce large-sized crystals while improving the crystal distribution uniformity of the product and appropriately improving the crystal morphology. A comparison of the crystal morphology of ropivacaine after reconstitution between the composition without metal ions (composition 6010) and the composition containing metal ions (composition 6064) is shown in FIG. 5.

### Example 4

### Study on Cholesterol

Using DMPC as a dispersant, the effects of different molar ratios of the active ingredient to cholesterol on dispersibility and crystal morphology were investigated. The formula amounts of ropivacaine, DMPC, and cholesterol were weighed according to Table 4-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). The D50 and D90 were measured with a laser particle size analyzer (OMEC, Topsizer). Furthermore, using the composition without cholesterol as a control, the settling time of ropivacaine crystals was observed after standing of the compositions, as shown in Table 4-2.

**Table 4-1. Compositions with different amounts of cholesterol**

| Composition No. | 6074 | 6075 | 6076 | 6077 | 6078 | 6079 | 6080 | 6081 | 6082 |
|---|---|---|---|---|---|---|---|---|---|
| | mg | mg | mg | mg | mg | mg | mg | mg | mg |
| ROP:cholesterol molar ratio | / | 28.2:1 | 11.3:1 | 9.1:1 | 7.0:1 | 5.6:1 | 3.8:1 | 2.8:1 | 1.9:1 |
| ROP | 200 | 200 | 200 | 200 | 200 | 200 | 200 | 200 | 200 |
| DMPC | 50 | 50 | 50 | 50 | 50 | 50 | 50 | 50 | 50 |
| CHO | 0 | 10 | 25 | 31 | 40 | 50 | 75 | 100 | 150 |
| 85%TBA | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL |

**Table 4-2. Dispersibility and crystal morphology**

| Composition No. | Crystal morphology | Particle size distribution | | Settling time |
|---|---|---|---|---|
| | | D50, µm | D90, µm | |
| 6074 | Irregular crystals | / | / | Less than 0.3 h |
| 6075 | Irregular crystals + rod-shaped crystals | 8.1 | 51.1 | More than 3 h |
| 6076 | Irregular crystals + rod-shaped crystals | 7.3 | 21.3 | More than 3 h |
| 6077 | Irregular crystals + rod-shaped crystals | 7.7 | 19.6 | More than 3 h |
| 6078 | Rod-shaped crystals | 8.2 | 18.9 | More than 3 h |
| 6079 | Rod-shaped crystals | 8.9 | 18.6 | More than 3 h |
| 6080 | Rod-shaped crystals | 7.7 | 17.1 | More than 3 h |
| 6081 | Rod-shaped crystals | 7.4 | 17.6 | More than 3 h |
| 6082 | Rod-shaped crystals | 7.2 | 26.6 | More than 3 h |

The results showed that: the composition without cholesterol exhibited irregular crystals, as shown in composition 6074 in FIG. 6; when the ropivacaine:cholesterol molar ratio was > 7:1, a small number of rod-shaped crystals could be seen, but there were still a large number of irregular crystals, as shown in composition 6076 in FIG. 6; when the ropivacaine:cholesterol molar ratio was ≤ 7:1, rod-shaped crystals were primarily formed, as shown in composition 6078 in FIG. 6. Moreover, the addition of cholesterol could prolong the settling time of ropivacaine crystals and enhance the physical stability of the reconstituted pharmaceutical solution.

### Example 5

### Study on Metal Ion Salts

### (1) Study on metal ion salt types

Using DMPC as a dispersant, the effects of monovalent and divalent metal ion salts on crystal morphology were investigated. The formula amounts of ropivacaine, DMPC, and different metal ion salts were weighed according to Tables 5-1 and 5-2 and added to Schott bottles, and then an aqueous *tert*-butanol solution was added. Each mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 5-1. Compositions containing monovalent metal ion salts**

| Composition No. | 6083 | 6084 | 6085 | 6086 |
|---|---|---|---|---|
| | mg | mg | mg | mg |
| ROP:metal ion molar ratio | 1:1.37 | 1:1.37 | 1:1.37 | 1:1.37 |
| ROP | 200 | 200 | 200 | 200 |
| DMPC | 50 | 50 | 50 | 50 |
| Sodium chloride | 58.44 | 0 | 0 | 0 |
| Potassium chloride | 0 | 74.55 | 0 | 0 |
| Potassium phosphate | 0 | 0 | 212.27 | 0 |
| Ammonium sulfate | 0 | 0 | 0 | 132.14 |
| 75%TBA | Added to make up to 10 mL | Added to make up to 10 mL | Added to make up to 10 mL | Added to make up to 10 mL |
| D50, µm | 9.1 | 10.8 | 9.6 | 13.2 |
| D90, µm | 41.1 | 46.3 | 30.6 | 31.0 |

**Table 5-2. Compositions containing divalent metal ion salts**

| Composition No. | 6087 | 6088 | 6089 | 6090 |
|---|---|---|---|---|
| | mg | mg | mg | mg |
| ROP:metal ion molar ratio | 1:1.37 | 1:1.37 | 1:1.37 | 1:1.37 |
| ROP | 200 | 200 | 200 | 200 |
| DMPC | 50 | 50 | 50 | 50 |
| Magnesium chloride | 203.3 | 0 | 0 | 0 |
| Magnesium sulfate | 0 | 246.47 | 0 | 0 |
| Calcium chloride | 0 | 0 | 147.02 | 0 |
| Barium chloride | 0 | 0 | 0 | 244.6 |
| 75%TBA | Added to make up to 10 mL | Added to make up to 10 mL | Added to make up to 10 mL | Added to make up to 10 mL |
| D50, µm | 10.7 | 12.8 | 13.2 | 10.1 |
| D90, µm | 40.7 | 40.7 | 41.9 | 37.0 |

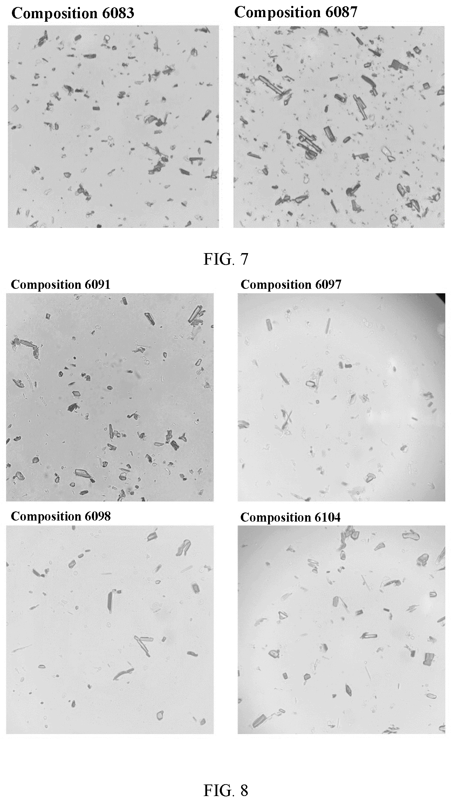

The results showed that both the monovalent and divalent metal ion salts could reduce large-sized crystals and appropriately improve the crystal morphology, making the crystals shorter and thicker, with the divalent metal ion salts exhibiting a more significant effect. The crystal morphology of ropivacaine in compositions 6083 and 6087 is shown in FIG. 7.

### (2) Study on amounts of metal ions

The effects of the ratio of the active ingredient to metal ions on crystal morphology were investigated separately, with sodium ions selected as monovalent metal ions and magnesium ions as divalent metal ions. Considering the injection route, sodium chloride and magnesium chloride were chosen for the study. The formula amounts of ropivacaine, DMPC, and different metal ion salts were weighed according to Tables 5-3 and 5-4 and added to Schott bottles, and then an aqueous *tert*-butanol solution was added. Each mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 5-3. Compositions containing monovalent metal ion salts**

| Composition No. | 6091 | 6092 | 6093 | 6094 | 6095 | 6096 | 6097 |
|---|---|---|---|---|---|---|---|
| | mg | mg | mg | mg | mg | mg | mg |
| ROP:NaCl molar ratio | 1:0.02 | 1:0.05 | 1:0.07 | 1:0.13 | 1:0.21 | 1:0.27 | 1:1.37 |
| ROP | 200 | 200 | 200 | 200 | 200 | 200 | 200 |
| DMPC | 50 | 50 | 50 | 50 | 50 | 50 | 50 |
| NaCl | 1 | 2 | 2.922 | 5.844 | 8.766 | 11.688 | 58.44 |
| 75%TBA | Added to make up to 10 mL | Added to make up to 10 mL | Added to make up to 10 mL | Added to make up to 10 mL | Added to make up to 10 mL | Added to make up to 10 mL | Added to make up to 10 mL |
| D50, µm | 12.5 | 12.6 | 14.2 | 19.2 | 17.1 | 16.3 | 17.3 |
| D90, µm | 47.2 | 47.3 | 51.8 | 73.0 | 65.6 | 64.9 | 63.8 |

**Table 5-4. Compositions containing divalent metal ion salts**

| Composition No. | 6098 | 6099 | 6100 | 6101 | 6102 | 6103 | 6104 |
|---|---|---|---|---|---|---|---|
| | mg | mg | mg | mg | mg | mg | mg |
| ROP:magnesium chloride molar ratio | 1:0.02 | 1:0.05 | 1:0.07 | 1:0.13 | 1:0.21 | 1:0.27 | 1:1.37 |
| ROP | 200 | 200 | 200 | 200 | 200 | 200 | 200 |
| DMPC | 50 | 50 | 50 | 50 | 50 | 50 | 50 |
| MgCl_{2·}6H₂O | 3.5 | 6.9 | 10.165 | 20.33 | 30.495 | 40.66 | 203.3 |
| 75%TBA | Added to make up to 10 mL | Added to make up to 10 mL | Added to make up to 10 mL | Added to make up to 10 mL | Added to make up to 10 mL | Added to make up to 10 mL | Added to make up to 10 mL |
| D50, µm | 10.5 | 9.9 | 7.5 | 10.7 | 13.2 | 13.9 | 19.2 |
| D90, µm | 39.6 | 37.8 | 37.2 | 43.3 | 55.2 | 53.4 | 55.4 |

The results showed that the addition of a certain amount of metal ions could reduce large-sized crystals and appropriately improve the crystal morphology. The morphology images of compositions 6091, 6097, 6098, and 6104 are shown in FIG. 8.

### (3) Study on method of introducing metal ions

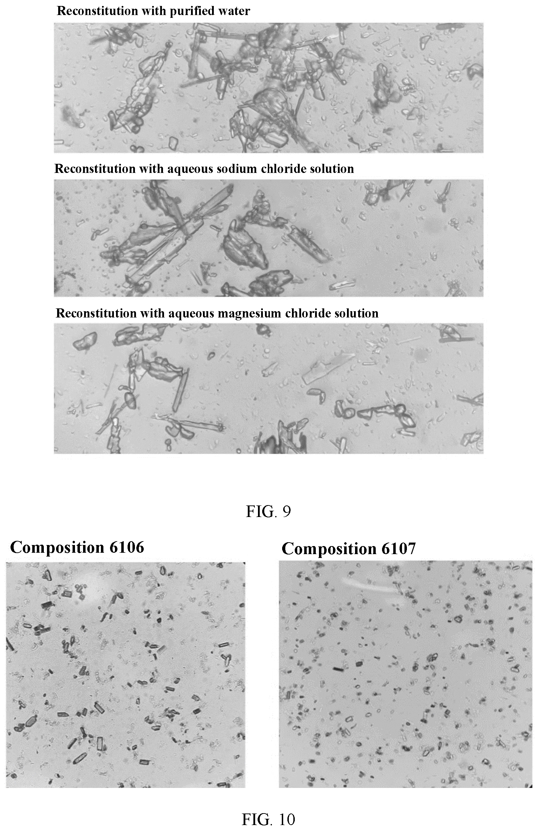

In order to further investigate the effects of metal ions, after freeze drying, the freeze-dried powder was reconstituted with a solution containing a metal ion salt to examine the influence of the method of adding metal ions on crystal morphology. An aqueous sodium chloride solution and an aqueous magnesium chloride solution were each prepared and added to composition 6105 (Table 5-5) at a molar ratio of ropivacaine:metal ions of 1:1.37. The mixture was shaken until the composition was uniformly dispersed. The crystal morphology of ropivacaine after reconstitution was then observed under a microscope (Olympus, CX41), as shown in FIG. 9.

**Table 5-5. Information on composition**

| Composition No. | 6105 |
|---|---|
| | mg |
| ROP | 400 |
| DMPC | 100 |
| 75%TBA | Added to make up to 20 mL |

**Table 5-6. Crystal morphology after reconstitution**

| Reconstitution medium | Crystal morphology |
|---|---|
| Purified water | Irregular crystals |
| Aqueous sodium chloride solution | Irregular crystals |
| Aqueous magnesium chloride solution | Irregular crystals |

The results showed that: the metal ions must be added in the initial formulation to achieve the effect of reducing large-sized crystals and appropriately improving the crystal morphology.

### Example 6

### Compositions Containing Cholesterol and Metal Ions

In the compositions, cholesterol and metal ions were simultaneously added to investigate their effects on crystal morphology. Sodium chloride and magnesium chloride were selected as metal ion salts for the study. The formula amounts of ropivacaine, DMPC, cholesterol, and a metal ion salt were weighed according to Table 6-1 and added to a Schott bottle (ropivacaine, cholesterol, and metal ions were added at a molar ratio of 1:0.18:1.37), and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 6-1. Information on compositions**

| Composition No. | 6106 | 6107 |
|---|---|---|
| | mg | mg |
| ROP:CHO:magnesium chloride molar ratio | 1:0.18:1.37 | 1:0.18:1.37 |
| ROP | 40 | 40 |
| DMPC | 10 | 10 |
| CHO | 10 | 10 |
| NaCl | 11.688 | 0 |
| MgCl_{2·}6H₂O | 0 | 40.56 |
| 75%TBA | Added to make up to 2 mL | Added to make up to 2 mL |
| D50, µm | 8.7 | 6.5 |
| D90, µm | 25.6 | 13.8 |

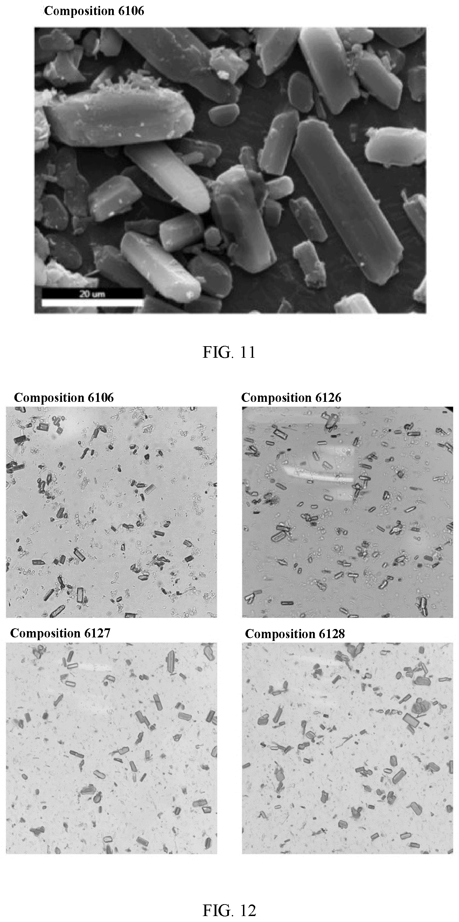

The results showed that the co-addition of cholesterol and metal ions exhibited a synergistic effect, enabling ropivacaine to form more regular rod-shaped crystals. The crystal morphology of ropivacaine in compositions 6106 and 6107 is shown in FIG. 10. The crystals of composition 6106 were isolated and then imaged using a scanning electron microscope (FEI, Quanta FEG 250, USA), revealing three-dimensional regular rod-shaped crystals, as shown in FIG. 11.

With the molar ratio of ropivacaine to cholesterol fixed, the effects of different amounts of metal ions on crystal morphology were investigated. The formula amounts of ropivacaine, DMPC, cholesterol, and a metal ion salt were weighed according to Tables 6-2 and 6-3 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 6-2. Information on compositions**

| Composition No. | 6108 | 6109 | 6110 | 6111 | 6112 | 6133 | 6106 | 6115 | 6116 |
|---|---|---|---|---|---|---|---|---|---|
| | mg | mg | mg | mg | mg | mg | mg | mg | mg |
| ROP:NaCl molar ratio | 1:0.02 | 1:0.05 | 1:0.07 | 1:0.13 | 1:0.21 | 1:0.27 | 1:1.37 | 1:1.6 | 1:2 |
| ROP | 200 | 200 | 200 | 200 | 200 | 200 | 200 | 200 | 200 |
| DMPC | 50 | 50 | 50 | 50 | 50 | 50 | 50 | 50 | 50 |
| CHO | 50 | 50 | 50 | 50 | 50 | 50 | 50 | 50 | 50 |
| NaCl | 1 | 2 | 2.922 | 5.844 | 8.766 | 11.688 | 58.44 | 70.8 | 85.4 |
| 75%TBA | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL | Added to make up to 10 mL |
| D50, µm | 10.5 | 9.7 | 10.4 | 10.3 | 10.0 | 12.4 | 8.7 | 8.2 | 7.9 |
| D90, µm | 32.7 | 27.8 | 44.2 | 34.7 | 35.9 | 46.1 | 25.6 | 23.0 | 20.3 |

**Table 6-3. Information on compositions**

| Composition No. | 6117 | 6118 | 6107 | 6120 | 6121 |
|---|---|---|---|---|---|
| | mg | mg | mg | mg | mg |
| ROP:magnesium chloride molar ratio | 1:0.27 | 1:0.82 | 1:1.37 | 1:1.6 | 1:2 |
| ROP | 200 | 200 | 200 | 200 | 200 |
| DMPC | 50 | 50 | 50 | 50 | 50 |
| CHO | 50 | 50 | 50 | 50 | 50 |
| MgCl₂ 6H₂O | 40.55 | 121.7 | 202.8 | 237.5 | 295 |
| 75%TBA | Added to make up to 10 mL | Added to make up to 10 mL | Added to make up to 10 mL | Added to make up to 10 mL | Added to make up to 10 mL |
| D50, µm | 8.9 | 5.1 | 6.5 | 7.0 | 7.0 |
| D90, µm | 30.4 | 10.1 | 13.8 | 15.5 | 16.1 |

The results showed that: when the molar ratio of ropivacaine to cholesterol was fixed and a certain amount of metal ions was added, the ropivacaine crystals were consistent with those in FIG. 10.

With the molar ratio of ropivacaine to metal ions fixed, the effects of different amounts of cholesterol on crystal morphology were investigated. The formula amounts of ropivacaine, DMPC, cholesterol, and a metal ion salt were weighed according to Table 6-4 and added to a Schott bottle, and then an aqueous TBA solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 6-4. Information on compositions**

| Composition No. | 6122 | 6123 | 6124 | 6125 | 6106 |
|---|---|---|---|---|---|
| | mg | mg | mg | mg | mg |
| ROP:cholesterol | 28.2:1 | 11.3:1 | 9.1:1 | 7.0:1 | 5.6:1 |
| ROP | 200 | 200 | 200 | 200 | 200 |
| DMPC | 50 | 50 | 50 | 50 | 50 |
| CHO | 10 | 25 | 31 | 40 | 50 |
| NaCl | 58.44 | 58.44 | 58.44 | 58.44 | 58.44 |
| 75% TBA | Added to make up to 10 mL | Added to make up to 10 mL | Added to make up to 10 mL | Added to make up to 10 mL | Added to make up to 10 mL |
| D50, µm | 10.1 | 8.5 | 9.4 | 7.6 | 8.7 |
| D90, µm | 32.1 | 27.3 | 31.9 | 21.1 | 25.6 |

The results showed that: when the molar ratio of ropivacaine to metal ions was fixed and the molar ratio of ropivacaine to cholesterol was ≤ 7:1, the ropivacaine crystals were consistent with those in FIG. 10.

### Example 7

### Compositions with Different Types and Amounts of Dispersants

### (1) Compositions with different types of dispersants

In the compositions, cholesterol and metal ions were simultaneously added; the type of dispersant was altered to investigate its effect on crystal morphology. Sodium chloride was selected as a metal ion salt for the study. The formula amounts of ropivacaine, a dispersant, cholesterol, and a metal ion salt were weighed according to Table 7-1 and added to a Schott bottle, and then a *tert*-butanol/water solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 7-1. Compositions with different types of dispersants**

| Composition No. | 6106 | 6126 | 6127 | 6128 |
|---|---|---|---|---|
| | mg | mg | mg | mg |
| ROP | 40 | 40 | 40 | 40 |
| DMPC | 10 | 0 | 0 | 0 |
| SPC | 0 | 10 | 0 | 0 |
| TW20 | 0 | 0 | 10 | 0 |
| TW80 | 0 | 0 | 0 | 10 |
| CHO | 10 | 10 | 10 | 10 |
| NaCl | 11.6 | 11.6 | 11.6 | 11.6 |
| 75% TBA | Added to make up to 2 mL | Added to make up to 2 mL | Added to make up to 2 mL | Added to make up to 2 mL |
| D50,µm | 8.7 | 9.7 | 11.1 | 8 |
| D90,µm | 25.6 | 22.1 | 27.1 | 21.5 |

The results showed that: the crystal morphology of ropivacaine in all the above compositions after reconstitution was rod-shaped, as shown in FIG. 12, indicating that the type of dispersant did not affect the crystal morphology.

### (2) Compositions with different amounts of dispersants

In the compositions, cholesterol and metal ions were simultaneously added; DMPC and polysorbate 20 were used as dispersants to investigate the effect of the amount of dispersant on crystal morphology. Sodium chloride was selected as a metal ion salt for the study. The formula amounts of ropivacaine, a dispersant, cholesterol, and a metal ion salt were weighed according to Tables 7-2 to 7-4 and added to a Schott bottle, and then a *tert*-butanol/water solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 7-2. Compositions with different amounts of dispersant**

| Composition No. | 6153 | 6155 | 6156 | 6157 | 6158 | 6159 |
|---|---|---|---|---|---|---|
| | mg | mg | mg | mg | mg | mg |
| ROP:dispersant molar ratio | 50:1 | 30:1 | 10:1 | 5:1 | 2:1 | 1:1 |
| ROP | 200 | 200 | 200 | 200 | 200 | 200 |
| DMPC | 10 | 16.5 | 49 | 99 | 250 | 475 |
| CHO | 50 | 50 | 50 | 50 | 50 | 50 |
| 75% TBA | Added to make up to 10 mL | Added to make up to 10 mL | Added to make up to 10 mL | Added to make up to 10 mL | Added to make up to 10 mL | Added to make up to 10 mL |
| D50,µm | 12.6 | 10.1 | 9.1 | 8.2 | 5.4 | 6.5 |
| D90,µm | 31.7 | 25.2 | 24.6 | 29.1 | 20.3 | 27.4 |

**Table 7-3. Compositions with different amounts of dispersant**

| Composition No. | 6166 | 6167 | 6168 | 6169 |
|---|---|---|---|---|
| | mg | mg | mg | mg |
| ROP:dispersant molar ratio | 10:1 | 2:1 | 10:1 | 2:1 |
| ROP | 200 | 200 | 200 | 200 |
| DMPC | 50 | 250 | 50 | 250 |
| CHO | 50 | 50 | 150 | 150 |
| 85% TBA | Added to make up to 10 mL | Added to make up to 10 mL | Added to make up to 10 mL | Added to make up to 10 mL |
| D50,µm | 7.1 | 6.6 | 12.4 | 7.7 |
| D90,µm | 17.6 | 37.7 | 30.3 | 18.3 |

**Table 7-4. Compositions with different amounts of dispersant**

| Composition No. | 6160 | 6161 | 6162 | 6163 | 6164 | 6165 |
|---|---|---|---|---|---|---|
| | mg | mg | mg | mg | mg | mg |
| ROP:dispersant molar ratio | 50:1 | 30:1 | 10:1 | 5:1 | 2:1 | 1:1 |
| ROP | 200 | 200 | 200 | 200 | 200 | 200 |
| TW20 | 7.5 | 12.5 | 40 | 75 | 195 | 400 |
| CHO | 50 | 50 | 50 | 50 | 50 | 50 |
| 75% TBA | Added to make up to 10 mL | Added to make up to 10 mL | Added to make up to 10 mL | Added to make up to 10 mL | Added to make up to 10 mL | Added to make up to 10 mL |
| D50,µm | 10.5 | 11.8 | 12.0 | 12.6 | 11.7 | 9.9 |
| D90,µm | 41.6 | 48.5 | 43.4 | 45.9 | 49.0 | 36.6 |

The results showed that: the crystal morphology of ropivacaine in all the above compositions after reconstitution was rod-shaped, which was consistent with that in FIG. 12, indicating that the amount of dispersant within the above molar ratio range did not affect the crystal morphology.

### Example 8

### Study on Different Solvent Ratios

In the compositions, cholesterol and metal ions were simultaneously added; the *tert*-butanol/water ratio was altered to investigate its effect on crystal morphology. For the study, sodium chloride was selected as a metal ion salt, and DMPC was selected as a dispersant. The formula amounts of ropivacaine, DMPC, cholesterol, and sodium chloride were weighed according to Table 8-1 and added to a Schott bottle, and then a *tert*-butanol/water solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 8-1. Compositions with different solvent ratios**

| Composition No. | 6106-1 | 6106-2 | 6133 | 6129 | 6129-1 |
|---|---|---|---|---|---|
| | mg | mg | mg | mg | mg |
| ROP | 40 | 40 | 40 | 40 | 40 |
| DMPC | 10 | 10 | 10 | 10 | 10 |
| CHO | 10 | 10 | 10 | 10 | 10 |
| NaCl | 11.6 | 11.6 | 2.3376 | 2.3376 | 2.3376 |
| Aqueous TBA solution | 65% TBA, added to make up to 2 mL | 70% TBA, added to make up to 2 mL | 75% TBA, added to make up to 2 mL | 80% TBA, added to make up to 2 mL | 85% TBA, added to make up to 2 mL |
| D50, µm | 7.7 | 12.1 | 12.4 | 7 | 7.8 |
| D90, µm | 24.1 | 35.4 | 46.1 | 16.7 | 20.0 |

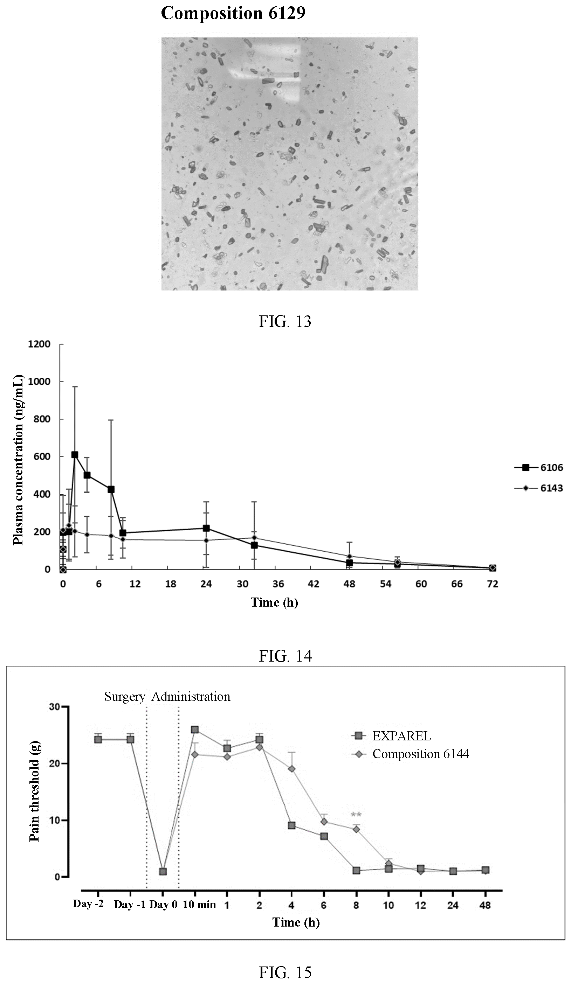

The results showed that: the compositions with different proportions of *tert*-butanol all exhibited regular rod-shaped crystals after reconstitution. The crystal morphology of ropivacaine in composition 6129 is shown in FIG. 13.

### Example 9

### Ropivacaine May Optionally Be Obtained Directly by Basification of Pharmaceutically Acceptable Salt of Ropivacaine During Formulation

If a salt is selected as the active ingredient, the crystal morphology can be improved by the addition of a metal ion-containing base, such as sodium hydroxide, potassium hydroxide, sodium bicarbonate, etc. The formula amounts of ropivacaine hydrochloride, DMPC, cholesterol, and a metal ion salt were weighed according to Table 9-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41). Furthermore, the D50 and D90 were measured using a laser particle size analyzer (OMEC, Topsizer).

**Table 9-1. Information on compositions**

| Composition No. | 6130 | 6131 | 6132 |
|---|---|---|---|
| | mg | mg | mg |
| ROP:sodium hydroxide molar ratio | 1: 1.1 | 1: 1.1 | 1: 1.1 |
| Ropivacaine hydrochloride | 239.7 | 239.7 | 239.7 |
| SPC | 50 | 0 | 0 |
| DMPC | 0 | 50 | 50 |
| CHO | 50 | 50 | 50 |
| Sodium hydroxide | 32 | 32 | 0 |
| Sodium bicarbonate | 0 | 0 | 67.35 |
| Sodium chloride | 11.75 | 11.75 | 0 |
| 75% TBA | Added to make up to 10 mL | Added to make up to 10 mL | Added to make up to 10 mL |
| D50, µm | 7.7 | 6.2 | 9.7 |
| D90, µm | 19.7 | 15.1 | 26.2 |

The results showed that: the crystal morphology of ropivacaine after reconstitution was rod-shaped, which was consistent with that in FIG. 10.

### Example 10

### Compositions with Different Active Ingredients

The formula amounts of an active ingredient, DMPC, cholesterol, and sodium chloride were weighed according to Table 10-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of the active ingredient after reconstitution was then observed under a microscope (Olympus, CX41), which showed that all the crystals were irregular, failing to develop regular crystal forms.

**Table 10-1. Information on compositions**

| Composition No. | 6106 | 6134 | 6135 | 6136 | 6137 | 6138 |
|---|---|---|---|---|---|---|
| | mg | mg | mg | mg | mg | mg |
| Bupivacaine | 200 | 0 | 0 | 0 | 0 | 0 |
| Lidocaine | 0 | 200 | 0 | 0 | 0 | 0 |
| Tetracaine | 0 | 0 | 200 | 0 | 0 | 0 |
| Prilocaine | 0 | 0 | 0 | 200 | 0 | 0 |
| Procaine | 0 | 0 | 0 | 0 | 200 | 0 |
| Benzocaine | 0 | 0 | 0 | 0 | 0 | 200 |
| DMPC | 50 | 50 | 50 | 50 | 50 | 50 |
| CHO | 50 | 50 | 50 | 50 | 50 | 50 |
| NaCl | 58.44 | 58.44 | 58.44 | 58.44 | 58.44 | 58.44 |
| 75% TBA | Added to make up to 10 mL | Added to make up to 10 mL | Added to make up to 10 mL | Added to make up to 10 mL | Added to make up to 10 mL | Added to make up to 10 mL |

### Example 11

### Compositions Containing Different Lyoprotectants

The formula amounts of ropivacaine, DMPC, cholesterol, sodium chloride, and a lyoprotectant were weighed according to Table 11-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The crystal morphology of ropivacaine after reconstitution was then observed under a microscope (Olympus, CX41), and the results were all consistent with those in FIG. 10. Furthermore, the D90 values were measured using a laser particle size analyzer (OMEC, Topsizer), all ranging between 10 µm and 50 µm.

**Table 11-1. Compositions containing different lyoprotectants**

| Composition No. | 6139 | 6140 | 6141 | 6142 |
|---|---|---|---|---|
| | g | g | g | g |
| ROP | 1 | 1 | 1 | 1 |
| DMPC | 0.25 | 0.25 | 0.25 | 0.25 |
| CHO | 0.25 | 0.25 | 0.25 | 0.25 |
| Sodium chloride | 0.058 | 0.058 | 0.058 | 0.292 |
| Mannitol | 0 | 0 | 0 | 0.375 |
| Trehalose | 0.3 | 0 | 1 | 0 |
| Sucrose | 0 | 1 | 0 | 0 |
| 75% TBA | Added to make up to 50 mL | Added to make up to 50 mL | Added to make up to 50 mL | Added to make up to 50 mL |

### Example 12

### In-Vivo Administration of Compositions

### Pharmacokinetics in rats

SD rats weighing about 200 g were allowed free access to water and food throughout the experimental period. Each group was subjected to administration by subcutaneous injection. Information on the samples is shown in Table 12-1.

The formula amounts of ropivacaine, DMPC, cholesterol, and sodium chloride were weighed according to Table 12-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed to form a 20 mg/mL suspension. The D50 and D90 were measured using a laser particle size analyzer (OMEC, Topsizer).

Each SD rat was subjected to administration at a dose of 20 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, and 72 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The plasma concentration curves are shown in FIG. 14.

**Table 12-1. Formulations of compositions**

| Composition No. | 6106 | 6143 |
|---|---|---|
| | mg | mg |
| ROP | 200 | 200 |
| DMPC | 50 | 10 |
| CHO | 50 | 10 |
| Sodium chloride | 58.44 | 58.44 |
| 75%TBA | Added to make up to 10 mL | Added to make up to 10 mL |
| D50, µm | 8.1 | 14.2 |
| D90, µm | 22.1 | 58.3 |

The results showed that: the compositions of the present disclosure all had favorable sustained-release effects.

### Example 13

### Pharmaceutical Compositions with Different pH Values

Acidic/alkaline regulators were added to the pharmaceutical compositions, or buffers with different pH values were used for reconstitution to adjust the pH of the pharmaceutical solutions after reconstitution, thereby investigating the effect of pH on the free drug (the drug dissolved in the solution), the particle size of ropivacaine crystals, and sustained-release properties.

### (1) Pharmaceutical compositions containing acidic regulator

The formula amounts of ropivacaine, DMPC, cholesterol, sodium chloride, and maleic acid were weighed according to Table 13-1 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The pH, particle size distribution, and free drug differences of the compositions after reconstitution were investigated.

**Table 13-1. Pharmaceutical compositions containing different amounts of acidic regulator**

| Composition No. | 6133 | 6145 | 6146 | 6147 | 6144 | 6148 |
|---|---|---|---|---|---|---|
| | mg | mg | mg | mg | mg | mg |
| ROP:acid molar ratio | / | 1:0.012 | 1:0.024 | 1:0.035 | 1:0.059 | 1:0.083 |
| ROP | 200 | 200 | 200 | 200 | 200 | 200 |
| DMPC | 50 | 50 | 50 | 50 | 50 | 50 |
| CHO | 50 | 50 | 50 | 50 | 50 | 50 |
| NaCl | 11.688 | 11.688 | 11.688 | 11.688 | 11.688 | 11.688 |
| Maleic acid | 0 | 1 | 2 | 3 | 5 | 7 |
| 75%TBA | Added to make up to 10 mL | Added to make up to 10 mL | Added to make up to 10 mL | Added to make up to 10 mL | Added to make up to 10 mL | Added to make up to 10 mL |

**Table 13-2. pH, free drug, and particle size distribution**

| Composition No. | pH after reconstitution | Free drug | Particle size distribution (µm) | |
|---|---|---|---|---|
| | | mg/mL | D50 | D90 |
| 6133 | 8.3 | 0.16 | 9.0 | 30.7 |
| 6145 | 7.4 | 0.52 | 9.0 | 27.1 |
| 6146 | 7.1 | 0.94 | 8.7 | 25.2 |
| 6147 | 6.9 | 1.40 | 8.3 | 23.7 |
| 6144 | 6.7 | 2.19 | 7.5 | 23.8 |
| 6148 | 6.6 | 2.83 | 8.7 | 22.2 |

The results showed that: the pH affected the concentration of free drug after reconstitution, but did not affect the particle size distribution. Compositions 6133 and 6144 were subjected to *in-vitro* release investigation. The pharmaceutical solutions obtained after reconstitution were each added to a dialysis bag, placed in 200 mL of PBS buffer at pH 7.4, and shaken on a 37 °C shaker at 50 rpm. Samples were then collected, and the amount of drug release was measured. The results are shown in Table 13-3.

**Table 13-3. In-vitro release results**

| Composition No. | *In-vitro* release (%) | | | | |
|---|---|---|---|---|---|
| | 0.5h | 2.5h | 6h | 24h | 72h |
| 6133 | 2.7 | 9.1 | 14.3 | 39.4 | 83.3 |
| 6144 | 6.7 | 13.8 | 20.8 | 48.9 | 91.3 |

The results showed that: the addition of the acidic regulator to the compositions altered the concentration of the free drug in the compositions, resulting in increased drug release after 30 min.

### (2) Pharmaceutical compositions containing alkaline regulator

The formula amounts of ropivacaine hydrochloride, DMPC, cholesterol, sodium hydroxide, and sodium chloride were weighed according to Table 13-4 and added to a Schott bottle, and then an aqueous *tert*-butanol solution was added. The mixture was appropriately heated and stirred until complete dissolution, then filtered through a 0.22 µm filter membrane, aliquoted at 2 mL/vial, and subjected to freeze drying according to Table 1-3. 2 mL of normal saline was added to the freeze-dried powder, and the mixture was shaken until the powder was uniformly dispersed. The pH and free drug differences of the compositions after reconstitution were investigated.

**Table 13-4. Pharmaceutical compositions containing different amounts of alkaline regulator**

| Composition No. | 6149 | 6150 |
|---|---|---|
| | mg | mg |
| Ropivacaine hydrochloride:sodium hydroxide molar ratio | 1:1.10 | 1:1.06 |
| Ropivacaine hydrochloride | 240 | 240 |
| SPC | 50 | 50 |
| CHO | 50 | 50 |
| Sodium hydroxide | 32.07 | 30.61 |
| Sodium chloride | 11.69 | 13.74 |
| 75%TBA | Added to make up to 10 mL | Added to make up to 10 mL |

**Table 13-5. pH and free drug**

| Composition No. | pH after reconstitution | Free drug |
|---|---|---|
| | | mg/mL |
| 6149 | 8.4 | 0.10 |
| 6150 | 7.2 | 0.75 |

The results showed that: by adjusting the ratio of the ropivacaine salt to the alkaline regulator, the concentration of the free drug can be controlled, thereby enabling modulation of drug release.

### (3) Composition reconstituted with buffers with different pH values

Composition 6133 in Table 13-1 was reconstituted with buffers with pH values ranging from 5.0 to 7.4. The pH and free drug of the composition solutions were then investigated.

**Table 13-6. pH and free drug results**

| Reconstitution medium | pH after reconstitution | Free drug |
|---|---|---|
| | | mg/mL |
| pH 5.0 buffer | 6.0 | 10.37 |
| pH 5.8 buffer | 6.4 | 5.08 |
| pH 6.5 buffer | 6.7 | 2.74 |
| pH 7.0 buffer | 7.0 | 1.63 |
| pH 7.4 buffer | 7.4 | 0.81 |

The results showed that: when the composition was reconstituted with different pH buffers, the concentration of the free drug differed. By adjusting the pH of the buffer used for reconstitution, the concentration of the free drug can be controlled, thereby enabling modulation of drug release.

### (4) In-vivo administration of compositions with different pH values

### Pharmacokinetics in rats

SD rats weighing about 200 g were allowed free access to water and food throughout the experimental period. Each group was subjected to administration by subcutaneous injection. Information on the samples is shown in Table 13-7. Each SD rat was subjected to administration at a dose of 20 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, and 72 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 13-8.

**Table 13-7. Formulations of compositions**

| Composition No. | 6106 | 6151 |
|---|---|---|
| | mg | mg |
| ROP | 200 | 200 |
| DMPC | 50 | 50 |
| CHO | 50 | 50 |
| Sodium chloride | 58.44 | 58.44 |
| Maleic acid | 0 | 7 |
| 75%TBA | Added to make up to 10 mL | Added to make up to 10 mL |
| pH after reconstitution | 8.1 | 6.6 |
| D50, µm | 8.1 | 8.7 |
| D90, µm | 22.1 | 22.2 |

**Table 13-8. Differences in plasma concentration in early stage (0 h to 2 h)**

| Composition No. | C (ng/mL) | | | |
|---|---|---|---|---|
| | 0.25h | 0.5h | 1h | 2h |
| 6106 | 109±50 | 199±101 | 202±145 | 611±362 |
| 6151 | 637,183 | 504±181 | 721±284 | 672±316 |

The results showed that: changing the pH of the composition altered only the initial Cₘₐₓ, without affecting the overall sustained-release effect.

### Pharmacokinetics in dogs

Beagle dogs weighing about 10 kg were allowed free access to water and food throughout the experimental period. Each group was subjected to administration by subcutaneous injection. Information on the samples is shown in Table 13-9. Each Beagle dog was subjected to administration at a dose of 6 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, 72 h, 96 h, 120 h, 144 h, and 168 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 13-10.

**Table 13-9. Formulations of compositions**

| Composition No. | 6146 | 6144 |
|---|---|---|
| | mg | mg |
| ROP | 200 | 200 |
| DMPC | 50 | 50 |
| CHO | 50 | 50 |
| Sodium chloride | 11.688 | 11.688 |
| Maleic acid | 2 | 5 |
| 75%TBA | Added to make up to 10 mL | Added to make up to 10 mL |
| pH after reconstitution | 7.1 | 6.8 |
| D50, µm | 8.7 | 7.5 |
| D90, µm | 25.2 | 23.9 |

**Table 13-10. Differences in plasma concentration in early stage (0 h to 2 h)**

| Composition No. | C (ng/mL) | | | |
|---|---|---|---|---|
| | 0.25h | 0.5h | 1h | 2h |
| 6146 | 63±22 | 75±32 | 78±27 | 60±27 |
| 6144 | 287±49 | 271±54 | 174±34 | 108±22 |

The results showed that: by adjusting the pH of the composition, the early-stage drug release can be improved, elevating the plasma concentrations within the period of 0.25 h to 2 h, thereby achieving the purpose of rapid onset.

### Example 14

### Comparative Studies with the Prior Art

The long-acting sustained-release aqueous systems of ropivacaine currently disclosed in patent literature mainly include ordinary suspension systems, liposome systems, microspheres, etc. Due to the multiple limitations of microspheres, the applicant selected an ordinary suspension system and a liposome system for comparative studies.

### (1) Comparative study with ordinary suspension system

In the ordinary suspension systems commonly used in disclosed patent literature, Tween or poloxamer is used as a dispersant, sodium carboxymethyl cellulose is used as a suspending agent, and the crystal particle size is controlled by shearing. The preparation method was as follows:
Sodium carboxymethyl cellulose, sodium chloride, and poloxamer 188 were weighed and dissolved in 45 mL of water for injection. After complete dissolution, ropivacaine was weighed and added to the above solution. Subsequently, high-shear processing was performed at 8000 rpm for 10 min to fully disperse the ropivacaine. The volume was then made up to 45 mL with water for injection. The resulting solution was aliquoted at 2 mL/vial, designated as Comparative Example 1.

Example composition 6106 of the present disclosure and the composition of Comparative Example 1 were subjected to pharmacokinetic studies in rats and dogs. The results are as follows:

### Pharmacokinetic study in rats

SD rats weighing about 200 g were allowed free access to water and food throughout the experimental period. Each group was subjected to administration by subcutaneous injection. Information on the samples is shown in Table 14-1. Each SD rat was subjected to administration at a dose of 20 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, and 72 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 14-2.

**Table 14-1. Information on compositions**

| Composition | 6106-2 | Comparative Example 1 |
|---|---|---|
| | mg | mg |
| ROP | 40 | 900 |
| DMPC | 10 | 0 |
| CHO | 10 | 0 |
| Sodium chloride | 11.688 | 303 |
| Sodium carboxymethyl cellulose | 0 | 189 |
| Poloxamer 188 | 0 | 15 |
| 70%TBA | Added to make up to 2 mL | 0 |
| Purified water | 0 | Added to make up to 45 mL |
| D50/D90(µm) | 12.1/35.4 | 13.7/34.1 |

**Table 14-2. Pharmacokinetic parameters**

| Composition No. | Cₘₐₓ | AUC_{inf} | T_{1/2} |
|---|---|---|---|
| | ng/mL | hr*ng/mL | hr |
| 6106-2 | 491.8±223 | 8413.7±2036 | 12.9±7.5 |
| Comparative Example 1 | 850.8±458 | 7409.1±1110 | 9.4±6.8 |

### Pharmacokinetic study in dogs

Beagle dogs weighing about 10 kg were allowed free access to water and food throughout the experimental period. Each group was subjected to administration by subcutaneous injection. Information on the samples is shown in Table 14-3. Each Beagle dog was subjected to administration at a dose of 6 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, 72 h, 96 h, 120 h, 144 h, and 168 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 14-4.

**Table 14-3. Information on compositions**

| Composition | 6146 | Comparative Example 1 |
|---|---|---|
| | mg | mg |
| ROP | 100 | 900 |
| DMPC | 25 | 0 |
| CHO | 25 | 0 |
| Sodium chloride | 5.844 | 303 |
| Maleic acid | 1 | 0 |
| Sodium carboxymethyl cellulose | 0 | 189 |
| Poloxamer 188 | 0 | 15 |
| 75%TBA | Added to make up to 5 mL | 0 |
| Purified water | 0 | Added to make up to 45 mL |
| D50/D90(µm) | 8.7/25.2 | 13.7/34.1 |

**Table 14-4. Pharmacokinetic parameters**

| Composition No. | Cₘₐₓ | AUC_{inf} | T_{1/2} | Absorption fraction (deconvolution method) | |
|---|---|---|---|---|---|
| | ng/mL | hr*ng/mL | hr | 24h(%) | 48h(%) |
| 6146 | 146±112 | 6330 | 69.1±38.7 | 31.99 | 53.10 |
| Comparative Example 1 | 180±120 | 4900±1350 | 10.7±6.2 | 65.37 | 92.88 |

The results showed that: compared with the ordinary suspension system, the suspension injection of the present application had superior pharmacokinetic properties *in vivo,* for example, its T_{1/2} in rats and dogs exhibited certain improvement; during scale-up production, the suspension injection of the present application can be filtered and sterilized, providing high sterility assurance.

### (2) Comparative study with liposome system

Among the liposome systems disclosed in patents, multilamellar vesicles and multivesicular liposomes predominate. Multilamellar vesicles are in the clinical research stage, while multivesicular liposomes already have a marketed formulation, EXPAREL, with bupivacaine as the active ingredient. Multilamellar vesicles were prepared as Comparative Example 2 and Comparative Example 3 according to the following method:
The formula amounts of the active ingredient, DMPC, and cholesterol were weighed and added to a Schott bottle, and then an aqueous *tert*-butanol solution was added. The mixture was appropriately heated and stirred until complete dissolution, then filtered through a 0.22 µm filter membrane, aliquoted at 5 mL/vial, and subjected to freeze drying according to the following process: pre-freezing at -45 °C for 120 min, primary drying at 0 °C for 720 min, and secondary drying at 30 °C for 1080 min. 5 mL of normal saline was added to the freeze-dried powder, and the mixture was shaken until the powder was uniformly dispersed for comparative studies.

A pharmacokinetic study and a local safety comparison study were conducted in rats. Furthermore, a pharmacokinetic study in dogs and a pharmacodynamic study in guinea pigs were performed using EXPAREL as a control drug. The results are as follows:

### Pharmacokinetic study in rats

SD rats weighing about 200 g were allowed free access to water and food throughout the experimental period. Each group was subjected to administration by subcutaneous injection. Information on the samples is shown in Table 14-5. Each SD rat was subjected to administration at a dose of 20 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, and 72 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 14-6.

**Table 14-5. Information on compositions**

| Composition | 6106 | Comparative Example 2 |
|---|---|---|
| | mg | mg |
| ROP | 100 | 100 |
| DMPC | 25 | 170 |
| CHO | 25 | 50 |
| Sodium chloride | 29.22 | 0 |
| 75%TBA | Added to make up to 5 mL | Added to make up to 5 mL |

**Table 14-6. Pharmacokinetic parameters**

| Composition No. | Cₘₐₓ | AUC_{inf} | T_{1/2} |
|---|---|---|---|
| | ng/mL | hr*ng/mL | hr |
| 6106 | 491.8±223 | 8413.7±2036 | 12.9±7.5 |
| Comparative Example 2 | 446.3±137.7 | 4967.0±480.2 | 5.9±2.0 |

### Pharmacokinetic study in dogs

Beagle dogs weighing about 10 kg were allowed free access to water and food throughout the experimental period. The marketed formulation EXPAREL was used as a control group. Composition 6144 and EXPAREL were administered by subcutaneous injection. Information on the composition is shown in Table 14-7. Each Beagle dog was subjected to administration at a dose of 6 mg/kg. Blood samples of about 0.3 mL were collected from animals in each group at 0 h (before administration) and at 15 min, 30 min, 1 h, 2 h, 4 h, 8 h, 10 h, 24 h, 32 h, 48 h, 56 h, 72 h, 96 h, 120 h, 144 h, and 168 h after administration into EDTA-2K⁺ anticoagulant blood collection tubes. After the whole blood was centrifuged at 8000 rpm for 5 min, plasma was collected. Subsequently, the drug concentration in each plasma sample was measured by LC-MS/MS. The pharmacokinetic parameters are shown in Table 14-8.

**Table 14-7. Information on composition**

| Composition | 6144 |
|---|---|
| | mg |
| ROP | 200 |
| DMPC | 50 |
| CHO | 50 |
| Sodium chloride | 11.688 |
| Maleic acid | 5 |
| 75%TBA | Added to make up to 10 mL |

**Table 14-8. Pharmacokinetic parameters**

| Composition No. | Cₘₐₓ | AUC_{inf} | T_{1/2} | Absorption fraction (deconvolution method) | |
|---|---|---|---|---|---|
| | ng/mL | hr*ng/mL | hr | 24h(%) | 48h(%) |
| 6144 | 334±32 | 9750.0 | 63.4±36.5 | 46.68 | 67.83 |
| EXPAREL | 387±253 | 9860±1200 | 26.3±11.9 | 41.87 | 74.00 |

### Pharmacodynamic study in rats

The marketed multivesicular liposome formulation EXPAREL was used as a control group. Composition 6144 and EXPAREL were administered by subcutaneous injection. Information on the composition is shown in Table 14-7. The EXPAREL group was subjected to administration at 6.65 mg/animal, and composition 6144 was administered at 10 mg/animal (equivalent dose to EXPAREL).

Baseline pain threshold detection was performed on two days before surgery (Day -2 and Day -1) and on the day of surgery (Day 0). Subsequently, preparation of a post-incisional pain model was started. All rats were first subjected to induction anesthesia with 3.5% to 4% isoflurane in oxygen, followed by maintenance anesthesia with 1.5% to 2% isoflurane. The foot skin was prepared with a povidone-iodine solution and alcohol. Under sterile conditions, a 1 cm longitudinal incision was made forward at 0.5 cm posterior to the metatarsal of each rat using a No. 11 scalpel blade. The fascia and muscle were separated, the flexor muscle was elevated, and a longitudinal incision was made along the muscle. The wound was held open for 2 h with a sterile cotton swab, and then two interrupted stitches were made at the incision site using 3-0 silk sutures to close the skin. Finally, povidone-iodine was applied to the skin at the incision site. After the surgery was completed, the rats were returned to breeding cages for recovery. At 24 h post-surgery (designated as Day 1), the postoperative baseline pain threshold was measured before administration. Then, drug administration was performed, and the pain thresholds at different time points after the administration were measured. The results are shown in FIG. 15.

### Local safety study in rats

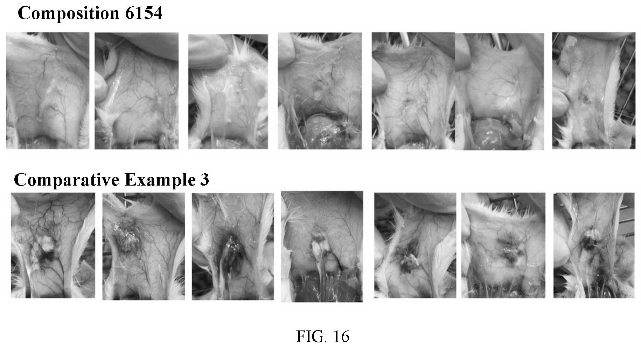

SD rats weighing about 200 g were allowed free access to water and food throughout the experimental period. Each group was subjected to administration by subcutaneous injection. Information on the samples is shown in Table 14-9. Each SD rat was subjected to administration at a dose of 20 mg/kg. Animals in each group were sacrificed at 72 h post-administration. The epidermis was dissected along the administration site, and the subcutaneous state and local irritation of the formulation were observed. The results are shown in FIG. 16.

**Table 14-9. Information on compositions**

| Composition | 6154 | Comparative Example 3 |
|---|---|---|
| | mg | mg |
| ROP | 200 | 240 |
| Ropivacaine hydrochloride | 0 | 68 |
| DMPC | 50 | 510 |
| CHO | 50 | 150 |
| Sodium chloride | 58.44 | 0 |
| Mannitol | 75 | 0 |
| TBA or an aqueous solution thereof | 75% TBA, added to make up to 10 mL | 100% TBA, added to make up to 10 mL |

**Table 14-11. Inflammation status**

| Composition No. | Inflammation status |
|---|---|
| 6154 | No inflammation in 5 animals (71.4%) |
| | Mild inflammation in 2 animals (28.6%) |
| Comparative Example 3 | Severe inflammation in 1 animal (14.3%) |
| | Moderate inflammation in 6 animals (85.7%) |

As can be seen from the above results, compared with the existing liposome system, the suspension injection of the present disclosure had superior pharmacokinetic properties *in vivo*, for example, its T_{1/2} in rats and dogs exhibited improvement; the local anesthetic effect (pain threshold) of the suspension injection was superior to that of the existing formulation EXPAREL; moreover, the suspension injection demonstrated fewer or milder inflammatory reactions and improved safety.

The embodiments of the present disclosure have been described above by way of examples. However, the claimed scope of the present disclosure is not limited to the exemplary embodiments described above. Any modification, equivalent replacement, improvement, etc., made by those skilled in the art without departing from the spirit and principle of the present disclosure shall fall within the claimed scope of the claims of the present application.

## Claims

1. A suspension of ropivacaine or a pharmaceutically acceptable salt thereof, comprising:
a) ropivacaine or the pharmaceutically acceptable salt thereof,
wherein in the suspension, ropivacaine or the pharmaceutically acceptable salt thereof is present in the form of particles, for example, in the form of crystals, preferably in the form of regular crystals, such as rod-shaped crystals or block-shaped crystals;
preferably, in the suspension, ropivacaine or the pharmaceutically acceptable salt thereof has a D90 particle size of less than or equal to 200 µm, or less than or equal to 150 µm, or less than or equal to 120 µm, or less than or equal to 100 µm; preferably, in the suspension, ropivacaine or the pharmaceutically acceptable salt thereof has a D90 particle size ranging from 10 µm to 90 µm, for example, 10 µm to 80 µm, preferably 10 µm to 70 µm, and more preferably 10 µm to 60 µm;
and/or the pharmaceutically acceptable salt of ropivacaine is an acidic salt of ropivacaine, i.e., an addition salt of ropivacaine with a pharmaceutically acceptable acid; for example, the acidic salt of ropivacaine comprises, but is not limited to, one or more selected from the following salts of ropivacaine: acetate, benzoate, benzenesulfonate, hydrobromide, camphorsulfonate, hydrochloride, citrate, edisylate, fumarate, glucoheptonate, gluconate, glucuronate, hydroiodide, isethionate, lactate, lactobionate, dodecylsulfate, malate, maleate, methanesulfonate, naphthoate, naphthalenesulfonate, nitrate, stearate, palmitate, myristate, laurate, oleate, oxalate, pamoate, phosphate, polygalacturonate, succinate, sulfate, sulfosalicylate, tartrate, toluenesulfonate, and trifluoroacetate.

2. The suspension according to claim 1, wherein the suspension optionally further comprises:
b) a pharmaceutically acceptable stabilizer;
preferably, the pharmaceutically acceptable stabilizer is selected from one or more of a long-chain alcohol (e.g., a C₁₈-C₃₀ long-chain alcohol) and a C₁₈-C₃₀ acid or a salt thereof;
preferably, the pharmaceutically acceptable stabilizer is selected from one or more of cholesterol, octadecanol, cholic acid or a salt thereof, deoxycholic acid or a salt thereof, taurodeoxycholic acid or a salt thereof, ursodeoxycholic acid or a salt thereof, chenodeoxycholic acid or a salt thereof, and glycocholic acid or a salt thereof;
preferably, the pharmaceutically acceptable stabilizer is selected from one or more of cholesterol, octadecanol, cholic acid, deoxycholic acid, taurodeoxycholic acid, ursodeoxycholic acid, chenodeoxycholic acid, glycocholic acid, sodium cholate, sodium deoxycholate, sodium taurodeoxycholate, sodium ursodeoxycholate, sodium chenodeoxycholate, and sodium glycocholate; preferably, the molar ratio of ropivacaine or the pharmaceutically acceptable salt thereof to the stabilizer ranges from 0.5:1 to 30:1, preferably 1:1 to 15:1, preferably 1:1 to 10:1, and more preferably 1:1 to 7:1;
and/or the suspension optionally further comprises:
c) a pharmaceutically acceptable dispersant;
preferably, the pharmaceutically acceptable dispersant is selected from one or more of a polysorbate (Tween), poloxamer, polyoxyethylene fatty acid ester, polyoxyethylene fatty acid ether, sucrose fatty acid ester, sodium dodecyl sulfate, macrogol 15 hydroxystearate (HS-15), and a phospholipid; preferably, the polysorbate is selected from one or more of polysorbate 80 (Tween 80) and polysorbate 20 (Tween 20);
preferably, the phospholipid is selected from one or more of DMPC (dimyristoylphosphatidylcholine), DPPC (dipalmitoylphosphatidylcholine), DLPC (dilauroylphosphatidylcholine), SPC (soybean phosphatidylcholine), EPC (egg phosphatidylcholine), DEPC (dierucoylphosphatidylcholine), DOPC (dioleoylphosphatidylcholine), and POPC (palmitoyloleoylphosphatidylcholine);
preferably, the molar ratio of ropivacaine or the pharmaceutically acceptable salt thereof to the pharmaceutically acceptable dispersant ranges from 50:1 to 1:1, preferably 40:1 to 2:1, more preferably 30:1 to 2:1, and more preferably 20:1 to 2:1;
and/or the suspension optionally further comprises:
d) a clinically acceptable diluent;
preferably, the clinically acceptable diluent comprises, but is not limited to, one or more of water for injection, a sodium chloride injection, a glucose injection, a Ringer's lactate solution, an aqueous sucrose solution, an aqueous xylitol solution, and a pH buffer;
preferably, the suspension is a suspension injection;
preferably, in the suspension, the concentration of ropivacaine or the pharmaceutically acceptable salt thereof ranges from about 0.1 mg/mL to about 300 mg/mL, preferably about 1 mg/mL to about 50 mg/mL, and more preferably about 2.5 mg/mL to about 40 mg/mL;
and/or the suspension optionally further comprises:
e) a pharmaceutically acceptable inorganic salt;
preferably, the pharmaceutically acceptable inorganic salt has a water solubility at 20 °C of greater than 10 g/100 mL;
preferably, the pharmaceutically acceptable inorganic salt is selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride;
preferably, the molar ratio of ropivacaine or the pharmaceutically acceptable salt thereof to the pharmaceutically acceptable inorganic salt is ≥ 1:0.02, and preferably ranges from 1:0.02 to 1:2.0; and/or the suspension optionally further comprises:
f) a pharmaceutically acceptable pH regulator;
preferably, the pH regulator is selected from an alkaline pH regulator and an acidic pH regulator; preferably, the alkaline pH regulator is selected from one or more of meglumine, tromethamine, sodium hydroxide, potassium hydroxide, sodium carbonate, sodium bicarbonate, magnesium hydroxide, calcium hydroxide, sodium phosphate, disodium hydrogen phosphate, potassium phosphate, dipotassium hydrogen phosphate, sodium citrate, sodium acetate, potassium acetate, ammonium acetate, triethylamine, ethanolamine, and diethanolamine, preferably one or more of sodium hydroxide, potassium hydroxide, magnesium hydroxide, sodium bicarbonate, and calcium hydroxide;
preferably, the acidic pH regulator is selected from one or more of citric acid, tartaric acid, maleic acid, malic acid, fumaric acid, succinic acid, hydrochloric acid, phosphoric acid, ascorbic acid, lactic acid, acetic acid, methanesulfonic acid, oxalic acid, sulfuric acid, glycine, sodium dihydrogen phosphate, and potassium dihydrogen phosphate, preferably one or more of citric acid, tartaric acid, maleic acid, malic acid, fumaric acid, succinic acid, hydrochloric acid, phosphoric acid, lactic acid, and acetic acid; preferably, the pH value of the suspension ranges from 6.0 to 9.0, preferably 6.0 to 8.5, preferably 6.0 to 8.0, and more preferably 6.0 to 7.5;
preferably, ropivacaine in the suspension may also be prepared *in situ,* for example, by reaction of a pharmaceutically acceptable salt of ropivacaine with a base, preferably by reaction of a pharmaceutically acceptable salt of ropivacaine with the alkaline pH regulator, wherein the pharmaceutically acceptable salt of ropivacaine has the definition described above;
preferably, when ropivacaine is prepared *in situ* as described above, the molar ratio of the pharmaceutically acceptable salt of ropivacaine to the alkaline pH regulator may range from 1:0.5 to 1:2.0, preferably 1:0.8 to 1:1.5, and preferably 1:1.0 to 1:1.2;
and/or the suspension optionally further comprises:
g) a pharmaceutically acceptable osmotic pressure regulator;
preferably, the osmotic pressure regulator is selected from one or more of sodium chloride, mannitol, lactose, glucose, sorbitol, and glycerol, preferably one or more of sodium chloride, glucose, sorbitol, and mannitol;
preferably, the osmotic pressure of the suspension injection is comparable to the physiological osmotic pressure;
and/or the suspension optionally further comprises:
h) a pharmaceutically acceptable lyoprotectant;
preferably, the pharmaceutically acceptable lyoprotectant is selected from one or more of mannitol, lactose, trehalose, sucrose, maltose, dextrin, stachyose, gelatin, pectin, sugar alcohol, sorbitol, proline, histidine, lysine, arginine, polyethylene glycol, and polyvinylpyrrolidone, preferably one or more of mannitol, lactose, trehalose, sucrose, and polyvinylpyrrolidone.

3. A solid composition of ropivacaine or a pharmaceutically acceptable salt thereof, comprising:
i) ropivacaine or the pharmaceutically acceptable salt thereof,
wherein in the solid composition, ropivacaine or the pharmaceutically acceptable salt thereof is present in the form of particles, for example, in the form of crystals, preferably in the form of rod-shaped or block-shaped crystals;
preferably, in the solid composition, ropivacaine or the pharmaceutically acceptable salt thereof has a D90 particle size of less than or equal to 200 µm, or less than or equal to 150 µm, or less than or equal to 120 µm, or less than or equal to 100 µm; preferably, in the solid composition, ropivacaine or the pharmaceutically acceptable salt thereof has a D90 particle size ranging from 10 µm to 90 µm, for example, 10 µm to 80 µm, preferably 10 µm to 70 µm, and more preferably 10 µm to 60 µm;
and/or the pharmaceutically acceptable salt of ropivacaine is an acidic salt of ropivacaine, i.e., an addition salt of ropivacaine with a pharmaceutically acceptable acid; for example, the acidic salt of ropivacaine comprises, but is not limited to, one or more selected from the following salts of ropivacaine: acetate, benzoate, benzenesulfonate, hydrobromide, camphorsulfonate, hydrochloride, citrate, edisylate, fumarate, glucoheptonate, gluconate, glucuronate, hydroiodide, isethionate, lactate, lactobionate, dodecylsulfate, malate, maleate, methanesulfonate, naphthoate, naphthalenesulfonate, nitrate, stearate, palmitate, myristate, laurate, oleate, oxalate, pamoate, phosphate, polygalacturonate, succinate, sulfate, sulfosalicylate, tartrate, toluenesulfonate, and trifluoroacetate.

4. The solid composition according to claim 3, wherein the solid composition optionally further comprises:
ii) a pharmaceutically acceptable stabilizer;
preferably, the pharmaceutically acceptable stabilizer is selected from one or more of a long-chain alcohol (e.g., a C₁₈-C₃₀ long-chain alcohol) and a C₁₈-C₃₀ acid or a salt thereof;
preferably, the pharmaceutically acceptable stabilizer is selected from one or more of cholesterol, octadecanol, cholic acid or a salt thereof, deoxycholic acid or a salt thereof, taurodeoxycholic acid or a salt thereof, ursodeoxycholic acid or a salt thereof, chenodeoxycholic acid or a salt thereof, and glycocholic acid or a salt thereof;
preferably, the pharmaceutically acceptable stabilizer is selected from one or more of cholesterol, octadecanol, cholic acid, deoxycholic acid, ursodeoxycholic acid, chenodeoxycholic acid, glycocholic acid, sodium cholate, sodium deoxycholate, and sodium taurodeoxycholate;
preferably, the molar ratio of ropivacaine or the pharmaceutically acceptable salt thereof to the stabilizer ranges from 0.5:1 to 30:1, preferably 1:1 to 15:1, preferably 1:1 to 10:1, and more preferably 1:1 to 7:1;
and/or the solid composition optionally further comprises:
iii) a pharmaceutically acceptable dispersant;
preferably, the pharmaceutically acceptable dispersant is selected from one or more of a polysorbate (Tween), poloxamer, polyoxyethylene fatty acid ester, polyoxyethylene fatty acid ether, sucrose fatty acid ester, sodium dodecyl sulfate, macrogol 15 hydroxystearate (HS-15), and a phospholipid; preferably, the polysorbate is selected from one or more of polysorbate 80 (Tween 80) and polysorbate 20 (Tween 20);
preferably, the phospholipid is selected from one or more of DMPC (dimyristoylphosphatidylcholine),
DPPC (dipalmitoylphosphatidylcholine), DLPC (dilauroylphosphatidylcholine), SPC (soybean phosphatidylcholine), EPC (egg phosphatidylcholine), DEPC (dierucoylphosphatidylcholine), DOPC (dioleoylphosphatidylcholine), and POPC (palmitoyloleoylphosphatidylcholine);
preferably, the molar ratio of ropivacaine or the pharmaceutically acceptable salt thereof to the pharmaceutically acceptable dispersant ranges from 50:1 to 1:1, preferably 40:1 to 2:1, more preferably 30:1 to 2:1, and more preferably 20:1 to 2:1;
and/or the solid composition optionally further comprises:
iv) a pharmaceutically acceptable inorganic salt;
preferably, the pharmaceutically acceptable inorganic salt has a solubility in water at 20 °C of greater than 10 g/100 mL;
preferably, the solid composition optionally comprises: an inorganic salt selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, preferably one or more of sodium chloride, potassium sulfate, magnesium chloride, magnesium sulfate, potassium chloride, and calcium chloride;
preferably, the molar ratio of ropivacaine or the pharmaceutically acceptable salt thereof to the pharmaceutically acceptable inorganic salt is ≥ 1:0.02, and preferably ranges from 1:0.02 to 1:2.0; and/or the solid composition optionally further comprises:
v) a pharmaceutically acceptable pH regulator;
preferably, the pH regulator is selected from an alkaline pH regulator and an acidic pH regulator;
preferably, the alkaline pH regulator is selected from one or more of meglumine, tromethamine, sodium hydroxide, potassium hydroxide, sodium carbonate, sodium bicarbonate, magnesium hydroxide, calcium hydroxide, sodium phosphate, disodium hydrogen phosphate, potassium phosphate, dipotassium hydrogen phosphate, sodium citrate, sodium acetate, potassium acetate, ammonium acetate, triethylamine, ethanolamine, and diethanolamine, preferably one or more of sodium hydroxide, potassium hydroxide, magnesium hydroxide, sodium bicarbonate, and calcium hydroxide;
preferably, the acidic pH regulator is selected from one or more of citric acid, tartaric acid, maleic acid, malic acid, fumaric acid, succinic acid, hydrochloric acid, phosphoric acid, ascorbic acid, lactic acid, acetic acid, methanesulfonic acid, oxalic acid, sulfuric acid, glycine, sodium dihydrogen phosphate, and potassium dihydrogen phosphate, preferably one or more of citric acid, tartaric acid, maleic acid, malic acid, fumaric acid, succinic acid, hydrochloric acid, phosphoric acid, lactic acid, and acetic acid; preferably, the solid composition forms a suspension upon reconstitution;
preferably, the pH value of the suspension ranges from 6.0 to 9.0, preferably 6.0 to 8.5, preferably 6.0 to 8.0, and more preferably 6.0 to 7.5;
and/or the solid composition optionally further comprises:
vi) a pharmaceutically acceptable osmotic pressure regulator;
preferably, the pharmaceutically acceptable osmotic pressure regulator is selected from one or more of sodium chloride, mannitol, lactose, glucose, sorbitol, and glycerol, preferably one or more of sodium chloride, glucose, sorbitol, and mannitol;
and/or the solid composition optionally further comprises:
vii) a pharmaceutically acceptable lyoprotectant;
preferably, the pharmaceutically acceptable lyoprotectant is selected from one or more of mannitol, lactose, trehalose, sucrose, maltose, dextrin, stachyose, gelatin, pectin, sugar alcohol, sorbitol, proline, histidine, lysine, arginine, polyethylene glycol, and polyvinylpyrrolidone, preferably one or more of mannitol, lactose, trehalose, sucrose, and polyvinylpyrrolidone.

5. A preparation method for a solid pharmaceutical composition, comprising: drying a solution comprising an active pharmaceutical ingredient to obtain the solid composition,
wherein in the solid pharmaceutical composition obtained after drying, the active pharmaceutical ingredient is present in the form of particles, for example, in the form of crystals, preferably in the form of rod-shaped crystals or block-shaped crystals;
preferably, the preparation method comprises: sterilizing, filtering, and drying a solution comprising an active pharmaceutical ingredient to obtain a solid composition of ropivacaine or a pharmaceutically acceptable salt thereof;
preferably, the drying is performed by a method including evaporation drying, freeze drying, and spray drying, preferably freeze drying;
preferably, the freeze drying comprises a pre-freezing procedure and a drying procedure;
preferably, the active pharmaceutical ingredient may be dissolved in a solvent to form the solution comprising the active pharmaceutical ingredient;
preferably, the solvent may be selected from an inorganic solvent, an organic solvent, and a mixture thereof suitable for dissolving the active pharmaceutical ingredient; for example, the solvent is water, an organic solvent, or a co-solvent system comprising water and an organic solvent, preferably one or more of water, methanol, ethanol, *n*-propanol, isopropanol, *n*-butanol, isobutanol, *tert*-butanol, acetone, acetonitrile, dichloromethane, and dimethyl sulfoxide, and more preferably water, ethanol, isopropanol, *tert*-butanol, or a co-solvent system comprising water and one or more organic solvents selected from the following: methanol, ethanol, *n*-propanol, isopropanol, *n*-butanol, isobutanol, *tert*-butanol, acetone, acetonitrile, dichloromethane, and dimethyl sulfoxide; for example, the co-solvent system is selected from a co-solvent system comprising water and one organic solvent selected from the following: ethanol, isopropanol, and *tert*-butanol;
preferably, in the co-solvent system of water and an organic solvent, the volume percentage of the organic solvent ranges from 1% to 99%, for example, 10% to 90%, preferably 50% to 90%;
preferably, the solid pharmaceutical composition may be selected from a solid composition of ropivacaine or a pharmaceutically acceptable salt thereof, preferably the solid composition of ropivacaine or the pharmaceutically acceptable salt thereof according to claim 3 or 4;
preferably, when the active pharmaceutical ingredient in the solution is a pharmaceutically acceptable salt, for example, a pharmaceutically acceptable acid addition salt or base addition salt, it may react with a pH regulator in the solution, thereby preparing *in situ* a corresponding free active ingredient, such as a free basic active ingredient or a free acidic active ingredient.

6. A preparation method for a suspension, comprising reconstituting the solid composition according to any one of claims 3 to 4 or the solid pharmaceutical composition according to claim 5, thereby obtaining the suspension,
wherein preferably, the preparation method comprises mixing the solid composition according to any one of claims 3 to 4 or the solid pharmaceutical composition according to claim 5 with a clinically acceptable diluent, thereby obtaining the suspension;
preferably, the clinically acceptable diluent comprises, but is not limited to, one or more of water for injection, a sodium chloride injection, a glucose injection, a Ringer's lactate solution, a sucrose solution, a xylitol solution, and a pH buffer.

7. Use of a pharmaceutically acceptable inorganic salt and/or a pharmaceutically acceptable stabilizer as a stabilizer for particles (e.g., crystals) of ropivacaine or a pharmaceutically acceptable salt thereof, preferably use of the pharmaceutically acceptable inorganic salt and/or the pharmaceutically acceptable stabilizer as a stabilizer for particles (e.g., crystals) of ropivacaine or a pharmaceutically acceptable salt thereof in the preparation method for the suspension of ropivacaine or the pharmaceutically acceptable salt thereof, or the solid composition of ropivacaine or the pharmaceutically acceptable salt thereof described above.

8. The use according to claim 7, wherein the pharmaceutically acceptable inorganic salt and/or the pharmaceutically acceptable stabilizer is used for promoting the stable presence of ropivacaine or the pharmaceutically acceptable salt thereof in the form of particles (e.g., crystals), preferably in the form of regular crystals, such as rod-shaped or block-shaped crystals, in the suspension or the solid composition.

9. The use according to claim 7 or 8, wherein the inorganic salt is selected from a pharmaceutically acceptable inorganic salt having a solubility in water of greater than 10 g/100 mL of water at 20 °C; preferably, the inorganic salt is selected from one or more of sodium chloride, sodium sulfate, potassium chloride, potassium sulfate, potassium phosphate, ammonium sulfate, ammonium chloride, magnesium chloride, magnesium sulfate, calcium chloride, and barium chloride, for example, selected from one or more of sodium chloride, potassium sulfate, magnesium chloride, magnesium sulfate, potassium chloride, and calcium chloride;
preferably, the pharmaceutically acceptable stabilizer is selected from a C₁₈-C₃₀ long-chain alcohol and a C₁₈-C₃₀ acid or a salt thereof, for example, selected from one or more of cholesterol, octadecanol, cholic acid, deoxycholic acid, ursodeoxycholic acid, chenodeoxycholic acid, glycocholic acid, sodium cholate, sodium deoxycholate, and sodium taurodeoxycholate.

10. Use of the suspension according to any one of claims 1 to 2 or the solid composition according to any one of claims 3 to 4 in the preparation of a medicament for producing anesthesia or relieving pain, or in the preparation of a medicament for treating and/or preventing pain,
wherein preferably, the suspension or the solid composition is administered around a nerve or to a surgical wound;
preferably, the administration is parenteral administration and/or topical administration;
preferably, the parenteral administration is selected from intravenous administration, intramuscular administration, subcutaneous injection, intraarticular injection, epidural administration, subarachnoid administration, incision perfusion administration, infiltration, and nerve block, preferably intramuscular administration, subcutaneous injection, intraarticular injection, epidural administration, subarachnoid administration, incision perfusion administration, infiltration, and nerve block; preferably, the pain is chronic postoperative pain or labor pain.
